# EUROPEAN PATENT APPLICATION

(11) **EP 4 756 010 A1**
(43) Date of publication of application: **10.06.2026**
(21) Application number: 24847791.1
(22) Date of filing: 11.06.2024
(51) Int. Cl.: C12N 1/20, A61K 35/74, A61K 45/06, A61P 35/00, A61P 37/04

(54) **STRAIN, CULTURE AND USE THEREOF**

(30) Priority: 28.07.2023 CN 202310946604
(71) Applicant: Moon (Guangzhou) Biotech Co., Ltd., Guangzhou, Guangdong 510535 (CN)
(72) Inventor: XIAN, Yibo, Guangzhou, Guangdong 510535 (CN); ZHAO, Yingying, Guangzhou, Guangdong 510535 (CN); ZHAO, Guozhen, Guangzhou, Guangdong 510535 (CN); CHEN, Zhipeng, Guangzhou, Guangdong 510535 (CN); JIANG, Xianzhi, Guangzhou, Guangdong 510535 (CN)
(74) Representative: Herzog IP Patentanwalts GmbH
(86) International application number: PCT/CN2024/098504
(87) International publication number: WO 2025/025828

(57) **Abstract**

The present disclosure discloses bacterial strains, cultures, and uses thereof. According to a first aspect of the present disclosure, a strain is provided. The strain can be a strain MNH19801 with an accession number GDMCC No. 61980 or a strain in which the 16S rRNA has an identity of at least 95% with 16S rRNA of the strain MNH19801 and/or a strain in which a genome has ANI of at least 87% with a genome of the strain MNH19801. The embodiments of the present disclosure disclose a novel strain belonging to the family *Lachnospiraceae.* This strain or a product derived from this strain demonstrates excellent efficacy in preventing or treating tumors, diabetes, or other inflammatory diseases.

## Description

### TECHNICAL FIELD

The present disclosure relates to the technical field of microbiology, and particularly relates to bacterial strains, culture, and uses thereof.

### BACKGROUND

The gut, the largest micro-ecological environment in the human body, is involved in various critical physiological processes such as nutrient absorption, energy metabolism, tissue and organ development, immune defense, and endocrine regulation in the human body. Thus, the gut is closely related to human health. In recent decades, leapfrog advancements have been achieved in the field of gut microecology, and the understanding of gut microecology by humans has been continuously deepened. The human gut harbors a vast community of symbiotic microorganisms. The collective genetic information carried by these symbiotic microorganisms is 50 to 100 times greater than the genetic information of the human. The collective genetic information of these symbiotic microorganisms, known as the gut microbiome, is often referred to as the "second genome" of the human. The gut microbiome constitutes the largest and most direct external environment of the human body, and plays an indispensable role in maintaining human health.

In recent years, with the rapid development of molecular biology, genomics, bioinformatics analysis technologies, high-throughput sequencing, and microbial culture techniques, the influence and effect of gut microbiota on intestinal and extraintestinal diseases have become increasingly well-defined. The gut microbiota works much like an organ with metabolic, immune, and endocrine functions. The gut microbiota can affect the human digestive, circulatory, and nervous systems, and is closely associated with the occurrence of various chronic diseases (such as diabetes, hypertension, cardiovascular diseases, and brain diseases) and tumors. Investigating the interrelationships of gut microbiota with human health and diseases not only constitutes significant scientific research, but also holds profound implications and values for clinical diagnosis, treatment, and even translational applications.

The imbalance in gut microbiota can increase the incidence of colorectal cancer, and metabolites produced by some gut microbes can directly mitigate carcinogenesis or inhibit tumorigenesis. In addition to intestinal cancers such colon cancer and rectal cancer, the gut microbiota affects breast cancer, liver cancer, etc. Increasing evidence has shown that the gut microbiota can influence the tumor formation and development, and effectively cooperate with cancer therapies. Continuing research focused on how to regulate immune and inflammatory responses induced by gut microbiota to enhance the efficacy of an anti-tumor drug without causing tumorigenesis. Resolving these issues is of great significance for future innovations and transformations in the cancer therapy field. Therefore, the exploration of new bacterial species and strains and the characterization of new functional chemical entities are required to develop novel therapeutic approaches. These efforts will contribute to accelerating the development of novel microbiome-directed therapies based on new bacterial species and functional chemicals.

### SUMMARY

The present disclosure is intended to solve at least one of the technical problems existing in the prior art. To this end, the present disclosure provides a novel strain. This strain can be used to treat tumors.

In a first aspect of the present disclosure, a bacterial strain is provided, where the strain is any one selected from A1) to A4):
A1) a bacterial strain, wherein the16S rRNA of the bacterial strain has an identity of at least 95%, 96%, 97%, 98%, 98.5%, 98.65%, 99%, 99.5%, 99.6%, 99.7%, 99.8%, 99.9%, 99.99%, or 100% with a sequence shown in SEQ ID NO: 1;
A2) bacterial strain MNH19801 having an Accession Number of GDMCC No. 61980;
A3) a bacterial strain, wherein the 16S rRNA of the bacterial strain has an identity of at least 95%, 96%, 97%, 98%, 98.5%, 98.65%, 99%, 99.5%, 99.6%, 99.7%, 99.8%, 99.9%, 99.99%, or 100% with the 16S rRNA of the strain described in A2); and
A4) a bacterial strain, wherein the genome of the bacterial strain has an average nucleotide identity (ANI) of at least 87%, 90%, 95%, 96%, 97%, 98%, 99%, 99.5%, 99.6%, 99.7%, 99.8%, 99.9%, 99.99%, or 100% with the genome of the strain described in any of A1) to A3); and/or, a bacterial strain, wherein the genome of the bacterial strain has an alignment score of at least 60%, 70%, 80%, 90%, 95%, 96%, 97%, 98%, or 99% with the genome of the strain described in any of A1) to A3).

In some embodiments, a sequence of the 16S rRNA of the strain described in the A2) is shown in SEQ ID NO: 1.

In some embodiments, the bacterial strain belongs to the family of *Lachnospiraceae.*

In some embodiments, the bacterial strain belongs to the genus of *Blautia* of the family of *Lachnospiraceae.*

In some embodiments, the strain is a novel species *(Blautia* sp.) of the genus of *Blautia* of the family of *Lachnospiraceae.*

In some embodiments, the strain is a novel species (*Oliverpabstia* sp.) of the genus of *Oliverpabstia* of the family of *Lachnospiraceae.*

In some embodiments, the strain is capable of producing acetic acid with a high yield.

The bacterial strain according to some embodiments exhibits at least the following beneficial effects:
The embodiments of the present disclosure disclose a novel strain belonging to the family *Lachnospiraceae.* This strain or a product derived from this strain demonstrates excellent efficacy in preventing or treating tumors or cancers, metabolic diseases, inflammatory or autoimmune diseases, infectious diseases, and central nervous system diseases.

In a second aspect of the present disclosure, a culture of the above-described strain is provided.

In some embodiments, the culture includes any one selected from C1) to C5):
C1) a fermentation medium for the strain; C2) a supernatant resulting from the fermentation medium for the strain; C3) a concentrate of C1) or C2); C4) an extract of C1) or C2); and C5) a dried product of any of C1) to C4).

In some embodiments, the fermentation medium is a liquid medium.

In some embodiments, the culture is produced by culturing the strain in the liquid medium under anaerobic culture conditions.

In some embodiments, the fermentation medium is an MM01 liquid medium, and/or the culturing is conducted at 35°C to 42°C, and/or the culturing is conducted for 24 h to 72 h, and/or a pH of the fermentation medium is 6 to 10.

In a third aspect of the present disclosure, a microbial agent including the above-described strain or a metabolite of the above-described strain or the above-described culture is provided.

In a fourth aspect of the present disclosure, a composition is provided, where the composition or a raw material for the composition includes the above-described strain or the above-described culture or the above-described microbial agent.

In some embodiments, the composition further includes at least one of an excipient, a diluent, and a carrier.

In some embodiments, the composition further includes an adjuvant.

In some embodiments, the adjuvant includes at least one of a lubricant, a wetting agent, an emulsifier, a suspension stabilizer, a preservative, a sweetener, and a flavoring agent.

In some embodiments, the composition is at least one of a drug, a nutritional supplement, a food, a food additive, a feed, and a feed additive.

In some embodiments, a dosage form of the composition is any one of a powder, a granule, a tablet, a capsule, a gel, a suspension, a drop, a drop pill, an injection, a suppository, an aerosol, an oral liquid, an ointment, an emulsion, and an irrigation solution.

In a fifth aspect of the present disclosure, a pharmaceutical composition is provided, where the pharmaceutical composition includes D1) and D2):
D1) the above-described strain or a metabolite of the above-described strain or the above-described culture or the above-described microbial agent; and
D2) a drug for combination therapy.

In some embodiments, D2) includes at least one selected from an anti-tumor drug, an anti-metabolic disease drug, an anti-inflammatory disease drug, an anti-autoimmune disease drug, an anti-infectious disease drug, and an anti-central nervous system disease drug.

In some embodiments, the anti-tumor drug includes at least one selected from a chemotherapeutic drug, a photosensitizer, a photothermal therapy agent, and an immunotherapeutic drug.

In some embodiments, the chemotherapeutic drug includes at least one selected from paclitaxel, camptothecin, 5-fluorouracil, cisplatin, doxorubicin, mitomycin, and epirubicin.

In some embodiments, the photosensitizer includes at least one selected from BODIPY, chlorin, and rose bengal.

In some embodiments, the photothermal therapy agent includes at least one selected from a noble metal nanoparticle, an organic polymer, a carbon-based nanomaterial, a magnetic nanomaterial, and a semiconductor nanomaterial.

In some embodiments, the immunotherapeutic drug includes at least one selected from an immune cell therapy drug and an immune checkpoint inhibitor.

In some embodiments, the immune cell therapy drug includes at least one selected from a T cell therapy drug, a tumor-infiltrating lymphocyte therapy drug, and a natural killer (NK) cell therapy drug.

In some embodiments, the immune checkpoint inhibitor includes an inhibitor targeting at least one of PD-1, PD-LI, PD-L2, CTLA-4, TIM-3, LAG-3, TIGIT, VISTA ,BTLA.

In some embodiments, the pharmaceutical composition includes 1 × 10³ to 10¹³ CFU/g of the strain.

In some embodiments, the pharmaceutical composition includes at least 1 × 10³ CFU/g, 2 × 10³ CFU/g, 5 × 10³ CFU/g, 1 × 10⁴ CFU/g, 2 × 10⁴ CFU/g, 5 × 10⁴ CFU/g, 1 × 10⁵ CFU/g, 2 × 10⁵ CFU/g, 5 × 10⁵ CFU/g, 1 × 10⁶ CFU/g, 2 × 10⁶ CFU/g, 5 × 10⁶ CFU/g, 1 × 10⁷ CFU/g, 2 × 10⁷ CFU/g, 5 × 10⁷ CFU/g, 1 × 10⁸ CFU/g, 2 × 10⁸ CFU/g, 5 × 10⁸ CFU/g, 1 × 10⁹ CFU/g, 2 × 10⁹ CFU/g, 5 × 10⁹ CFU/g, 1 × 10¹⁰ CFU/g, 2 × 10¹⁰ CFU/g, 5 × 10¹⁰ CFU/g, 1 × 10¹¹ CFU/g, 2 × 10¹¹ CFU/g, 5 × 10¹¹ CFU/g, 1 × 10¹² CFU/g, 2 × 10¹² CFU/g, 5 × 10¹² CFU/g, or 1 × 10¹³ CFU/g of the strain.

In some embodiments, the strain in the pharmaceutical composition includes a live bacterium, an attenuated bacterium, a killed bacterium, a lyophilized bacterium, or an irradiated bacterium.

In some embodiments, the pharmaceutical composition or the raw material for the pharmaceutical composition includes about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, or 80% of D1.

In some embodiments, the pharmaceutical composition or the raw material for the pharmaceutical composition includes 1% to 80%, 2% to 70%, 5% to 60%, 10% to 50%, or 20% to 40% of D1.

In some embodiments, the pharmaceutical composition further includes at least one of an excipient, a diluent, and a carrier.

In some embodiments, the pharmaceutical composition further includes a pharmaceutically acceptable adjuvant.

In some embodiments, the pharmaceutically acceptable adjuvant includes at least one of a lubricant, a wetting agent, an emulsifier, a suspension stabilizer, a preservative, a sweetener, and a flavoring agent.

In some embodiments, the pharmaceutical composition is in an infant-suitable dosage form, a child-suitable dosage form, or an adult-suitable dosage form.

In some embodiments, the pharmaceutical composition is in a dosage form for enteral administration or a dosage form for parenteral administration.

In some embodiments, the pharmaceutical composition is an oral preparation or an injection.

In some embodiments, a dosage form of the pharmaceutical composition is any one selected from a powder, a granule, a tablet, a capsule, a gel, a suspension, a drop, a drop pill, an injection, a suppository, an aerosol, an oral liquid, an ointment, an emulsion, and an irrigation solution.

In some embodiments, the strain is capable of at least partially proliferating in the intestine of a subject.

In some embodiments, the pharmaceutical composition further includes an instruction for use.

In a sixth aspect of the present disclosure, a pharmaceutical combination product is provided, including the following independent components:
a first product including the above-described strain or a metabolite of the above-described strain or the above-described culture or the above-described microbial agent; and
a second product including a drug for combination therapy.

In some embodiments, the drug for combination therapy includes at least one of an anti-tumor drug, an anti-metabolic disease drug, an anti-inflammatory disease drug, an anti-autoimmune disease drug, an anti-infectious disease drug, and an anti-central nervous system disease drug.

In some embodiments, the pharmaceutical combination product further includes a package insert.

In some embodiments, the anti-tumor drug includes at least one of a chemotherapeutic drug, a photosensitizer, a photothermal therapy agent, and an immunotherapeutic drug.

In some embodiments, the chemotherapeutic drug includes at least one of paclitaxel, camptothecin, 5-fluorouracil, cisplatin, doxorubicin, mitomycin, and epirubicin.

In some embodiments, the photosensitizer includes at least one of BODIPY, chlorin, and rose bengal.

In some embodiments, the photothermal therapy agent includes at least one of a noble metal nanoparticle, an organic polymer, a carbon-based nanomaterial, a magnetic nanomaterial, and a semiconductor nanomaterial.

In some embodiments, the immunotherapeutic drug includes at least one of an immune cell therapy drug and an immune checkpoint inhibitor.

In some embodiments, the immune cell therapy drug includes at least one of a T cell therapy drug, a tumor-infiltrating lymphocyte therapy drug, and an NK cell therapy drug.

In some embodiments, the immune checkpoint inhibitor includes an inhibitor targeting at least one of PD-1, PD-L1, PD-L2, CTLA-4, TIM-3, LAG-3, TIGIT, VISTA, and BTLA.

In some embodiments, the first product includes 1 × 10³ CFU/g to 1 × 10¹³ CFU/g of the strain.

In some embodiments, the first product includes at least 1 × 10³ CFU/g, 2 × 10³ CFU/g, 5 × 10³ CFU/g, 1 × 10⁴ CFU/g, 2 × 10⁴ CFU/g, 5 × 10⁴ CFU/g, 1 × 10⁵ CFU/g, 2 × 10⁵ CFU/g, 5 × 10⁵ CFU/g, 1 × 10⁶ CFU/g, 2 × 10⁶ CFU/g, 5 × 10⁶ CFU/g, 1 × 10⁷ CFU/g, 2 × 10⁷ CFU/g, 5 × 10⁷ CFU/g, 1 × 10⁸ CFU/g, 2 × 10⁸ CFU/g, 5 × 10⁸ CFU/g, 1 × 10⁹ CFU/g, 2 × 10⁹ CFU/g, 5 × 10⁹ CFU/g, 1 × 10¹⁰ CFU/g, 2 × 10¹⁰ CFU/g, 5 × 10¹⁰ CFU/g, 1 × 10¹¹ CFU/g, 2 × 10¹¹ CFU/g, 5 × 10¹¹ CFU/g, 1 × 10¹² CFU/g, 2 × 10¹² CFU/g, 5 × 10¹² CFU/g, or 1 × 10¹³ CFU/g of the strain.

In some embodiments, the strain in the first product includes a live bacterium, an attenuated bacterium, a killed bacterium, a lyophilized bacterium, or an irradiated bacterium.

In some embodiments, the first product and/or the second product further includes at least one of an excipient, a diluent, and a carrier.

In some embodiments, the first product and the second product independently further include at least one pharmaceutically acceptable adjuvant.

In some embodiments, the at least one pharmaceutically acceptable adjuvant includes at least one of a lubricant, a wetting agent, an emulsifier, a suspension stabilizer, a preservative, a sweetener, and a flavoring agent.

In some embodiments, the pharmaceutical combination product is in an infant-suitable dosage form, a child-suitable dosage form, or an adult-suitable dosage form.

In some embodiments, the first product and the second product are each independently in a dosage form for enteral administration or a dosage form for parenteral administration.

In some embodiments, the first product and the second product are each independently an oral preparation or an injection.

In some embodiments, the first product and the second product are each independently have a dosage form of any one of a powder, a granule, a tablet, a capsule, a gel, a suspension, a drop, a drop pill, an injection, a suppository, an aerosol, an oral liquid, an ointment, an emulsion, and an irrigation solution.

In some embodiments, the strain is capable of at least partially proliferating in an intestine of a subject.

In a seventh aspect of the present disclosure, the present disclosure provides a use of the above-described strain, the above-described culture, the above-described microbial agent, the above-described composition, the above-described pharmaceutical composition, or the above-described pharmaceutical combination product in preparation of a drug for preventing and/or treating a disease.

In some embodiments, the disease includes at least one of a tumor or cancer, a metabolic disease, an inflammatory or autoimmune disease, an infectious disease, and a central nervous system disease.

In some embodiments, the tumor or cancer includes at least one of a solid tumor, a soft tissue tumor, a hematopoietic tumor, a glandular tumor, and a metastatic tumor.

In some embodiments, the solid tumor includes at least one of head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer.

In some embodiments, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some embodiments, the hematopoietic tumor includes a tumor in at least one of blood, lymph, and bone marrow.

In some embodiments, the tumor or cancer includes a tumor or cancer caused by a bacterium or virus.

In some embodiments, the bacterium or virus includes at least one of hepatitis B virus, hepatitis C virus, human papillomavirus, Epstein-Barr (EB) virus, *Helicobacter pylori, and Fusobacterium nucleatum.*

In some embodiments, the metabolic disease includes a disease characterized by a metabolic disorder in at least one of an amino acid, an organic acid, a sugar, a fat, a purine, a pigment, blood ammonia, and a metal ion.

In some embodiments, the inflammatory or autoimmune disease includes at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome.

In some embodiments, the infectious disease includes a disease caused by an infection with at least one of a bacterium, a virus, and a fungus.

In some embodiments, the central nervous system disease includes at least one of stroke, schizophrenia, drug addiction, Alzheimer's disease, Parkinson's disease, anxiety disorder, and depression.

In some embodiments, preventing and/or treating a tumor is achieved through at least one of following (a) to (l): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of a PD-1 inhibitor; (f) preventing and/or inhibiting dissemination or metastasis of a tumor cell; (g) reducing resistance to an anti-tumor drug; (h) inhibiting an activity of a histone deacetylase (HDAC) through at least one of short-chain fatty acids (SCFAs) including acetic acid or acetate, propionic acid or propionate, butyric acid or butyrate, and valeric acid or valerate; (i) inhibiting an activity of HDAC and/or promoting a transcriptional activity of interferon-β (IFN-β); (j) modulating an immune system of a subject to play an immunomodulatory role; (k) promoting senescence or apoptosis of a tumor cell; and (l) inhibiting angiogenesis within a tumor tissue.

In an eighth aspect of the present disclosure, a use of the above-described strain, the above-described culture, the above-described microbial agent, the above-described composition, the above-described pharmaceutical composition, or the above-described pharmaceutical combination product in prevention and/or treatment of a disease.

In some embodiments, the disease includes at least one of a tumor or cancer, a metabolic disease, an inflammatory or autoimmune disease, an infectious disease, and a central nervous system disease.

In some embodiments, the tumor or cancer includes at least one of a solid tumor, a soft tissue tumor, a hematopoietic tumor, a glandular tumor, and a metastatic tumor.

In some embodiments, the solid tumor includes at least one of head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer.

In some embodiments, the hematopoietic tumor includes a tumor in at least one of blood, lymph, and bone marrow.

In some embodiments, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some embodiments, the tumor or cancer includes a tumor or cancer caused by a bacterium or virus.

In some embodiments, the bacterium or virus includes at least one of hepatitis B virus, hepatitis C virus, human papillomavirus, EB virus, *Helicobacter pylori, and Fusobacterium nucleatum.*

In some embodiments, the metabolic disease includes a disease characterized by a metabolic disorder in at least one of an amino acid, an organic acid, a sugar, a fat, a purine, a pigment, blood ammonia, and a metal ion.

In some embodiments, the inflammatory or autoimmune disease includes at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome.

In some embodiments, the infectious disease includes a disease caused by an infection with at least one of a bacterium, a virus, and a fungus.

In some embodiments, the central nervous system disease includes at least one of stroke, schizophrenia, drug addiction, Alzheimer's disease, Parkinson's disease, anxiety disorder, and depression.

In some embodiments, preventing and/or treating a tumor is achieved through at least one of following (a) to (k): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of a PD-1 inhibitor; (f) preventing and/or inhibiting dissemination or metastasis of a tumor cell; (g) reducing resistance to an anti-tumor drug; (h) inhibiting an activity of HDAC through at least one of the acetic acid or acetate, propionic acid or propionate, butyric acid or butyrate, and valeric acid or valerate in SCFA; (i) inhibiting an activity of HDAC and/or promoting a transcriptional activity of IFN-β; (j) modulating an immune system of a subject to play an immunomodulatory role; (k) promoting senescence or apoptosis of a tumor cell; and (l) inhibiting angiogenesis within a tumor tissue.

In a ninth aspect, the present disclosure provides a method for treating a disease, including administering an effective dose of the above-described strain, the above-described culture, the above-described microbial agent, the above-described composition, the above-described pharmaceutical composition, or the above-described pharmaceutical combination product to a subject.

In some embodiments, the disease includes at least one of a tumor or cancer, a metabolic disease, an inflammatory or autoimmune disease, an infectious disease, and a central nervous system disease.

In some embodiments, the tumor or cancer includes at least one of a solid tumor, a soft tissue tumor, a hematopoietic tumor, a glandular tumor, and a metastatic tumor.

In some embodiments, the solid tumor includes at least one of head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer.

In some embodiments, the hematopoietic tumor includes a tumor in at least one of blood, lymph, and bone marrow.

In some embodiments, the tumor or cancer includes at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia.

In some embodiments, the tumor or cancer includes a tumor or cancer caused by a bacterium or virus.

In some embodiments, the bacterium or virus includes at least one of hepatitis B virus, hepatitis C virus, human papillomavirus, EB virus, *Helicobacter pylori, and Fusobacterium nucleatum.*

In some embodiments, the metabolic disease includes a disease characterized by a metabolic disorder in at least one of an amino acid, an organic acid, a sugar, a fat, a purine, a pigment, blood ammonia, and a metal ion.

In some embodiments, the inflammatory or autoimmune disease includes at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome.

In some embodiments, the infectious disease includes a disease caused by an infection with at least one of a bacterium, a virus, and a fungus.

In some embodiments, the central nervous system disease includes at least one of stroke, schizophrenia, drug addiction, Alzheimer's disease, Parkinson's disease, anxiety disorder, and depression.

In some embodiments, preventing and/or treating a tumor is achieved through at least one of following (a) to (k): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of a PD-1 inhibitor; (f) preventing and/or inhibiting dissemination or metastasis of a tumor cell; (g) reducing resistance to an anti-tumor drug; (h) inhibiting an activity of HDAC through at least one of acetic acid or acetate, propionic acid or propionate, butyric acid or butyrate, and valeric acid or valerate in SCFA; (i) inhibiting an activity of HDAC and/or promoting a transcriptional activity of IFN-β; (j) modulating an immune system of a subject to play an immunomodulatory role; (k) promoting senescence or apoptosis of a tumor cell; and (l) inhibiting angiogenesis within a tumor tissue.

In some embodiments, the strain, the culture, the microbial agent, the composition, the pharmaceutical composition, or the pharmaceutical combination product is administered at least once daily.

In some embodiments, when the strain, the culture, the microbial agent, the composition, the pharmaceutical composition, or the pharmaceutical combination product is administered twice or more daily, the strain, the culture, the microbial agent, the composition, the pharmaceutical composition, or the pharmaceutical combination product is administered at a same dose.

In some embodiments, when the strain, the culture, the microbial agent, the composition, the pharmaceutical composition, or the pharmaceutical combination product is administered twice or more daily, the strain, the culture, the microbial agent, the composition, the pharmaceutical composition, or the pharmaceutical combination product is administered at different doses.

In some embodiments, a route for the administering includes at least one of oral administration, sublingual administration, nasal administration, rectal administration, inhalation administration, transdermal administration, intraperitoneal injection, subcutaneous injection, and intramuscular injection.

In a tenth aspect, the present disclosure provides a method for preparing the above-described culture is provided, including following step:
inoculating the above-described strain into a fermentation medium to produce a culture.

In some embodiments, the method further includes centrifuging the culture and collecting a supernatant resulting from the fermentation medium.

In some embodiments, the method further includes concentrating the fermentation medium or the supernatant resulting from the fermentation medium through at least one of evaporation, lyophilization, dialysis, extraction, and membrane separation to produce a concentrate.

In some embodiments, the method further includes subjecting the fermentation medium or the supernatant resulting from the fermentation medium to at least one of extraction and solvent extraction to produce an extract.

In some embodiments, the method further includes drying any one of the fermentation medium, the supernatant resulting from the fermentation medium, the concentrate, and the extract to produce a dried product.

Additional aspects and advantages of the present disclosure will be partially described below, partially become apparent from the description, or understood through the practice of the present disclosure.

### BRIEF DESCRIPTION OF THE DRAWINGS

FIG. 1 shows a colony morphology of a strain MNH 19801 cultured on an anaerobic blood agar plate for 48 h;
FIG. 2 shows a microscopic morphology of the strain MNH 19801 at a scale bar of 2 µm;
FIG. 3 shows a microscopic morphology of the strain MNH 19801 at a scale bar of 10 µm;
FIG. 4 shows a microscopic morphology of the strain MNH 19801 under Gram staining;
FIG. 5 shows a microscopic morphology of the strain MNH 19801 under spore staining;
FIG. 6 shows tolerance test results of the strain MNH 19801 to different pH levels;
FIG. 7 shows tolerance test results of the strain MNH 19801 to NaCl at different concentrations;
FIG. 8 shows tolerance test results of the strain MNH 19801 to a bile salt at different concentrations;
FIG. 9 shows a phylogenetic tree constructed based on 16S rRNA of the strain MNH 19801 and other highly-similar strains;
FIG. 10 shows regulatory effects of the strain MNH 19801 on an immune activity of macrophages;

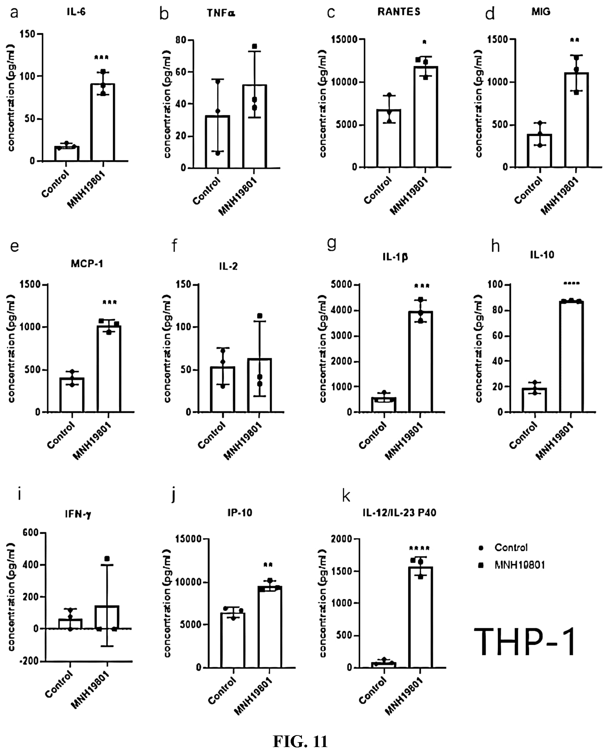

FIG. 11 shows regulatory effects of the strain MNH 19801 on the secretion of cytokines by macrophages that are determined by cytometric bead array (CBA) assay, where a to k show changes in the secretion of interleukin-6 (IL-6), tumor necrosis factor-alpha (TNF-α), regulated on activation, normal T-cell expressed and secreted (RANTES), monokine induced by gamma-interferon (MIG), monocyte chemoattractant protein-1 (MCP-1), interleukin-2 (IL-2), interleukin-1 beta (IL-1β), interleukin-10 (IL-10), interferon-gamma (IFN-γ), interferon-gamma-inducible protein 10 (IP-10), and interleukin-12/interleukin-23 subunit p40 (IL-12/IL-23P40) by macrophages, respectively; and **: p < 0.01, ***: p < 0.001, and ****: p < 0.0001;
FIG. 12 shows an enhancing effect of the strain MNH19801 on a transcriptional activity of IFN-β;
FIG. 13 shows an inhibitory effect of the strain MNH19801 on an activity of HDAC;
FIG. 14 shows analysis results for the strain MNH 19801 in activating immune systems of tumor-bearing mice, where A shows a CD3/CD45 ratio and B shows a CD8/CD45 ratio; and in both A and B, A5 indicates an MNH 19801 experimental group and CT indicates a control group;
FIG. 15 shows tumor volume curves of mice in a control group and an experimental group, where data is expressed as mean ± standard deviation (mean ± SD); statistical analysis is performed using two-way ANOVA (2way ANOVA) in combination with Sidak's test for multiple comparisons; and significant differences are indicated by *: *p < 0.05 and **p < 0.01, and a non-significant difference is not marked;
FIG. 16 shows response rates of mice in a control group (A) and an experimental group (B) at an experimental endpoint, where data is expressed as a tumor volume (mm³) per individual mouse;
FIG. 17 shows tumor volume change curves of mice in various groups over different intervention time points, where data is expressed as mean tumor volume ± standard deviation (mm³); at an experimental endpoint, statistical analysis is performed using Two-Way ANOVA in combination with Dunnett's test for multiple comparisons; and compared to a negative control group, statistical significance is defined as follows: no significant difference (ns): p ≥ 0.05; *: p < 0.05; **: p < 0.01; ***: p < 0.001; and ****: p < 0.0001;
FIG. 18 shows tumor volumes of mice in various groups at an endpoint, where data is expressed as mean tumor volume ± standard deviation (mm³); at an experimental endpoint, statistical analysis is performed using Two-Way ANOVA in combination with Dunnett's test for multiple comparisons; and compared to a negative control group, statistical significance is defined as follows: no significant difference (ns): p ≥ 0.05; *: p < 0.05; **: p < 0.01; ***: p < 0.001; and ****: p < 0.0001;
FIG. 19 shows tumor inhibition rate/tumor growth inhibition (TGI) results of mice at an experimental endpoint, where data is expressed as mean tumor volume ± standard deviation (mm³); at an experimental endpoint, statistical analysis is performed using Two-Way ANOVA in combination with Dunnett's test for multiple comparisons; and compared to a control group, statistical significance is defined as follows: no significant difference (ns): p ≥ 0.05; *: p < 0.05; **: p < 0.01; ***: p < 0.001; and ****: p < 0.0001;
FIG. 20 shows response rates of mice in a negative control group (A), a PD-1 group (B), and a combination therapy group (C) at an experimental endpoint, where data is expressed as a tumor volume (mm³) per individual mouse;
FIG. 21 shows tumor volume change curves of mice in a negative control group, a PD-1 group, and a combination therapy group over different intervention time points in Example 12;
FIG. 22 shows flow cytometry results of molecules MHCII, CD80, and CD86 on the surface of dendritic cells (DCs) in Example 13; and
FIG. 23 shows quantitative polymerase chain reaction (qPCR) detection results of the expression of M2 markers CD206 and Arg1 by RAW264.7 cells in Example 14.

### DETAILED DESCRIPTION

The concept of the present disclosure and the technical effects produced by the present disclosure will be described clearly and completely below with reference to embodiments, such that the objectives, features, and effects of the present disclosure can be fully understood. Apparently, the described examples are merely some rather than all of the examples of the present disclosure. All other examples obtained by those skilled in the art based on the examples of the present disclosure without creative efforts should fall within the protection scope of the present disclosure.

The embodiments of the present disclosure are described in detail below. The described embodiments are exemplary and only intended to explain the present disclosure, but should not be construed as a limitation to the present disclosure.

In the description of the present disclosure, the term "several" means one or more, the term "a plurality of" means two or more, the terms such as "greater than", "less than", and "exceeding" are construed to exclude the listed number, the terms "no less than", "no more than", and "within" are construed to include the listed number, and the term "about" indicates a range of ±20%, 10%, 8%, 5%, 4%, 3%, 2%, 1%, 0.5%, 0.2%, 0.1%, etc., of the listed number. The "first" and "second" in the description are merely intended to distinguish technical features, rather than to indicate or imply relative importance or implicitly indicate a number of the indicated technical features or implicitly indicate a sequential order of the indicated technical features.

In the description of the present disclosure, the description with reference to the terms "one embodiment", "some embodiments", "exemplary embodiments", "examples", "specific examples", or "some examples" means that specific features, structures, materials, or characteristics described in combination with the embodiments or examples are included in at least one embodiment or example of the present disclosure. In this specification, the schematic representations of the above terms do not necessarily refer to a same embodiment or example. In addition, the specific features, structures, materials, or characteristics described may be combined in any suitable manner in any one or more embodiments or examples.

A first aspect of the embodiments of the present disclosure provides a strain MNH 19801. This strain was deposited in the Guangdong Microbial Culture Collection Center (GDMCC) under an accession number GDMCC No. 61980 on October 27, 2021, with a taxonomic designation of *Lachnospiraceae* sp. MNH19801. The Guangdong Microbial Culture Collection Center (GDMCC) is located at the Guangdong Institute of Microbiology, Guangdong Academy of Sciences on No. 59 Building, No. 100, Xianliezhong Road, Guangzhou.

The term "strain" refers to a member of a bacterial species that possesses a genetic characteristic distinguishing the member from closely related members of the same bacterial species. The genetic characteristic may include the complete or partial absence of at least one gene, the complete or partial absence of at least one regulatory region (such as a promoter, a terminator, a riboswitch, and a ribosome binding site), the presence of at least one recombinant gene, the presence of at least one mutated gene, the presence of at least one exogenous gene (a gene from another species), the presence of at least one non-natural plasmid, the presence of at least one antibiotic resistance cassette, or a combination thereof. A genetic characteristic distinguishing between different strains can be identified through polymerase chain reaction (PCR) amplification, and optionally, DNA sequencing can be subsequently conducted for a genomic region of interest or the entire genome. In the case where a strain (compared to another strain of a same species) gains or loses antibiotic resistance, or gains or loses biosynthetic capacity (such as an auxotrophic strain), the strains can be distinguished through selection or counter-selection using an antibiotic or by meeting a specific nutrition/metabolite requirement.

It is known in the art that strains can be classified and identified through traditional taxonomic approaches and molecular biology techniques. The traditional taxonomic approaches include, for example, cell morphology observation, Gram staining, flagella staining, various metabolic assays, etc. The molecular biology techniques include ribosomal RNA sequencing, whole genome sequencing-based techniques, etc. The strain involved in the embodiments of the present disclosure has been identified as a novel genus in the family *Lachnospiraceae* based on the following taxonomic characteristics:

Bacterial cells do not have spores and flagella, and are non-motile, short rod-shaped, and Gram-positive.

When this strain is inoculated on an anaerobic blood agar plate and cultured under anaerobic conditions (for example, optionally, this strain is cultured anaerobically at 37°C for 48 h), visible colonies are generated on the anaerobic blood agar plate. These colonies are circular with regular edges, white, and opaque, and have a diameter of approximately 2 mm. No secretions are produced around these colonies.

A growth temperature range for this strain is 30°C to 42°C, and an optimal growth temperature for this strain is approximately 37°C. This strain can grow in a pH range of 6 to 10, with an optimal growth pH of 7.0. This strain cannot grow at a NaCl concentration of more than 2%. This strain can survive and grow in a bile salt concentration range of 0% to 0.25%, but the growth of this strain trends to be weakened with the increase in bile salt concentration. This strain cannot grow under aerobic conditions, but grows well under anaerobic conditions. Thus, this strain belongs to obligate anaerobic bacteria.

Given the obligate anaerobic characteristic of this strain, an API 20A test is conducted for this strain. Test results show that this strain is sensitive to at least one of antibiotics including gentamicin, erythromycin, chloramphenicol, tetracycline, penicillin, ampicillin, and ceftriaxone, and is resistant to at least one of antibiotics including ciprofloxacin, trimethoprim/sulfamethoxazole, and lincomycin.

In some embodiments, the strain or a variant thereof exhibits the characteristic of high-yield acetic acid production. The high-yield acetic acid production refers to an acetic acid yield of more than or equal to 200 µg/g in short-chain fatty acid (SCFA) analysis. Specifically, the acetic acid yield is higher than or equal to 300 µg/g, higher than or equal to 400 µg/g, higher than or equal to 500 µg/g, higher than or equal to 600 µg/g, higher than or equal to 700 µg/g, higher than or equal to 800 µg/g, higher than or equal to 900 µg/g, higher than or equal to 1,000 µg/g, higher than or equal to 1,100 µg/g, higher than or equal to 1,200 µg/g, higher than or equal to 1,300 µg/g, or higher than or equal to 1,400 µg/g. For example, after the strain or the variant thereof is inoculated into a Trypticase Soy Broth (TSB) liquid medium and cultured anaerobically at 37°C for 48 h, an acetic acid yield detected after the collection of bacterial cells through centrifugation is higher than or equal to 300 µg/g, higher than or equal to 400 µg/g, higher than or equal to 500 µg/g, higher than or equal to 600 µg/g, higher than or equal to 700 µg/g, higher than or equal to 800 µg/g, higher than or equal to 900 µg/g, higher than or equal to 1,000 µg/g, higher than or equal to 1,100 µg/g, higher than or equal to 1,200 µg/g, higher than or equal to 1,300 µg/g, or higher than or equal to 1,400 µg/g.

Further, according to sequencing results for 16S rRNA of the strain MNH19801, a sequence of the 16S rRNA is shown in SEQ ID NO: 1. 16S rRNA is a ribosomal RNA in prokaryotes. The 16S rRNA gene includes variable regions and conserved regions. The conserved regions are shared by all bacteria, while the variable regions vary among different bacteria to different degrees. By comparing sequences of 16S rRNA genes in bacteria and calculating evolutionary distances based on nucleotide divergences, a phylogenetic tree can be plotted.

When an identity between 16S rRNA gene sequences of two strains is lower than 98.65%, these two strains can be classified as belonging to different species (refer to Kim, M., Oh, H.-S., Park, S.-C., & Chun, J. (2014). Towards a taxonomic coherence between average nucleotide identity and 16S rRNA gene sequence similarity for species demarcation of prokaryotes. INTERNATIONAL JOURNAL OF SYSTEMATIC AND EVOLUTIONARY MICROBIOLOGY, 64(Pt 2), 346-351, and Liu, C., Du, M.-X., Abuduaini, R., Yu, H.-Y., Li, D.-H., Wang, Y.-J., Liu, S.-J. (2021). Enlightening the taxonomy darkness of human gut microbiomes with a cultured biobank. Microbiome, 9(1), P23; Pablo Yarza,Uniting the classification of cultured and uncultured bacteria and archaea using 16S rRNA gene sequences,Microbiology,volume 12, P635-P645,2014).

When an identity between 16S rRNA gene sequences of two strains is lower than 95%, these two strains can be classified as belonging to different genera ("A genus definition for Bacteria and Archaea based on genome relatedness and taxonomic affiliation", R.A. Barco, bioRxiv(doi: https://doi.org/10.1101/392480);Evaluation of 16S rRNA gene sequencing for species and strain-level microbiome analysis, Jethro S. Johnson, Nature Communications 10, Article number: 5029 (2019)).

Therefore, in some embodiments of the present disclosure, variants of the strain MNH19801 based on the aforementioned 16S rRNA sequence are provided. These variants has an identity of at least 95%, 96%, 97%, 98%, 98.5%, 98.65%, 99%, 99.5%, 99.6%, 99.7%, 99.8%, 99.9%, 99.99%, or 100% with the strain MNH19801 under the accession number GDMCC No. 61980 in terms of 16S rRNA sequences. Consequently, these variants are of the same species as the strain MNH19801 or of different species from the strain MNH19801 in a same genus. Thus, it can be reasonably inferred that these strains, either of a same species or different species in a same genus, possess similar biological activities. In some other embodiments, these variants carry a 16S rRNA sequence having an identity of at least 95%, 96%, 97%, 98%, 98.5%, 98.65%, 99%, 99.5%, 99.6%, 99.7%, 99.8%, 99.9%, 99.99%, or 100% with the sequence shown in SEQ ID NO: 1.

The "identity" between sequences of two nucleic acid molecules can be determined using a known computer algorithm, such as at least one of a GCG program package, BLASTN, and FASTA. Other commercial or publicly available programs may include, for example, DNAStar "MegAlign" program.

Moreover, with the rapid development of second-generation and third-generation sequencing technologies, the identification of strains based on whole genome sequencing has become feasible, which can enhance the accuracy of identification results of strains. An average nucleotide identity (ANI) of two bacterial genomes refers to a similarity of homologous genes between the two bacterial genomes. In the field of bacterial taxonomy, it is widely believed that an ANI value of 98.65% or more is required to define two strains as belonging to a same species( Kim, M., Oh, H.-S., Park, S.-C., & Chun, J. (2014). Towards a taxonomic coherence between average nucleotide identity and 16S rRNA gene sequence similarity for species demarcation of prokaryotes. INTERNATIONAL JOURNAL OF SYSTEMATIC AND EVOLUTIONARY MICROBIOLOGY, 64(Pt 2), 346-351, and Liu, C., Du, M.-X., Abuduaini, R., Yu, H.-Y, Li, D.-H., Wang, Y.-J., Liu, S.-J. (2021). Enlightening the taxonomy darkness of human gut microbiomes with a cultured biobank. Microbiome, 9(1), P23; Pablo Yarza, Uniting the classification of cultured and uncultured bacteria and archaea using 16S rRNA gene sequences, Microbiology,volume 12, P635-P645, 2014).

When an identity between 16S rRNA gene sequences of two strains is lower than 95%, these two strains can be classified as belonging to different genera ("A genus definition for Bacteria and Archaea based on genome relatedness and taxonomic affiliation", R.A. Barco, bioRxiv(doi: https://doi.org/10.1101/392480);Evaluation of 16S rRNA gene sequencing for species and strain-level microbiome analysis, Jethro S. Johnson, Nature Communications 10 , Article number: 5029 (2019)).

With the above method, those skilled in the art can determine whether an isolated strain belongs to the family *Lachnospiraceae* and is a novel strain of the same species as the strain MNH19801 or is of a different species from the strain MNH19801 in a same genus. For example, when the isolated strain has an ANI value of at least 87% such as at least 87%, 90%, 95%, 95.5%, 96%, 96.5%, 97%, 97.5%, 98%, 98.5%, 99%, 99.5%, 99.6%, 99.7%, 99.8%, 99.9%, 99.99%, or 100% with the strain under the accession number GDMCC No. 61980, it can be determined that the isolated strain belongs to the same genus or the same species as the strain under the accession number GDMCC No. 61980.

There have been various mature calculation tools for ANI, such as the locally operated software Jspecies (/jspecies) and Gegenees (/documentation.html) and the online calculation tools including ANI calculator (http:/enveomics.gatech.edu/), EzGenome (/ezgenome/ani), and ANItools. Therefore, an ANI value between the isolated strain and the strain MNH19801 can be calculated using any of the aforementioned locally operated software or the online calculation tools and other tools or methods known in the art.

Therefore, in some embodiments of the present disclosure, variants of the strain MNH19801 based on the aforementioned genome are provided. These variants has an ANI value of at least 95%, 96%, 97%, 98%, 98.5%, 98.65%, 99%, 99.5%, 99.6%, 99.7%, 99.8%, 99.9%, 99.99%, or 100% with the strain MNH19801 under the accession number GDMCC No. 61980 or any of the aforementioned other variants in terms of genomes. Consequently, these variants are of the same species as the strain MNH19801 or of different species from the strain MNH19801 in a same genus. Thus, it can be reasonably inferred that these strains, either of a same species or different species in a same genus, possess similar biological activities. The determination can also be conducted based on an alignment fraction of corresponding genomes. For example, a variant having an alignment fraction of at least 60%, 70%, 80%, 90%, 95%, 96%, 97%, 98%, or 99% with the strain MNH19801 under the accession number GDMCC No. 61980 or any of the aforementioned other variants also possesses an identical or similar biological activity.

In some embodiments, based on the alignment results of both 16S rRNA and ANI, a variant of the strain carries 16S rRNA having an identity of at least 95%, 96%, 97%, 98%, 98.5%, 98.65%, 99%, 99.5%, 99.6%, 99.7%, 99.8%, 99.9%, 99.99%, or 100% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801.

For example, a variant of the strain carries 16S rRNA having an identity of at least 95% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 96% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 97% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 98% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 98.5% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 98.65% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 99% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 99.5% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 99.6% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 99.7% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 99.8% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 99.9% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801;
A variant of the strain carries 16S rRNA having an identity of at least 99.99% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801; or
A variant of the strain carries 16S rRNA having an identity of 100% with the 16S rRNA of MNH19801, and carries a genome having an ANI value of at least 87%, 90%, 95%, 96%, 97%, 98%, 99%, 99.5%, 99.6%, 99.7%, 99.8%, 99.9%, 99.99%, or 100% with the genome of MNH19801.

The term "variant" includes any bacteria derived from the strain MNH19801, such as bacteria generated through natural mutation or recombination (for example, a mutation resulting from cell proliferation or cell division), resistance screening, or mutation or recombination based on any other mechanism including radiation, a virus, a transposon, or a mutagenic chemical. The variant also includes other strains isolated from natural environments that share the same or similar taxonomic characteristics and properties with the strain MNH19801. The variant generally includes strains in the family *Lachnospiraceae* that belong to the same genus as but of different species from MNH19801, or belong to the same species as but exhibit different phenotypic variations from MNH19801.

A second aspect of the embodiments of the present disclosure relates to a culture of the strain. The term "culture" refers to a product obtained by culturing an isolated strain in a fermentation medium. The fermentation medium may be a natural or artificial medium, and can be, for example, a solid medium or a liquid medium. In some embodiments, the fermentation medium includes a peptone, such as at least one of meat peptone, casein peptone, whey peptone, and soybean peptone. The fermentation medium may further include other inorganic salts or organic substances to promote bacterial growth.

In some embodiments, the culture can be a fermentation medium for the strain; a supernatant produced by centrifuging the fermentation medium; a concentrate produced by subjecting the fermentation medium or the supernatant to at least one of evaporation, lyophilization, dialysis, extraction, and membrane separation; an extract produced by subjecting the fermentation medium or the supernatant to at least one of extraction and solvent extraction (for example, using water or an organic solvent); or a dried product produced by drying any one of the fermentation medium, the supernatant, the concentrate, and the extract. Further, in some specific embodiments, the extract may be produced through extraction targeting one or more specific components, such as extraction targeting components with specific molecular weight ranges, specific solubility, specific isoelectric points, etc.; and may also be produced through extraction not targeting one or more specific components. A specific extraction process is as follows: At least one of methanol, ethanol, chloroform, water, an acid, and an alkali is used to conduct extraction in a specific process sequence at a specified temperature. For example, extraction may be conducted in 75 v/v% ethanol at 60°C to 80°C for 1 min to 10 min, or in methanol at -30°C to -10°C for 1 min to 10 min, or in a methanol/chloroform/water solution at -40°C to -20°C for 30 min to 60 min, or in deionized water at 95°C for 1 min to 10 min, or in a perchloric acid, trichloroacetic acid, hydrochloric acid, or sodium hydroxide solution at 0°C to 4°C, so as to extract corresponding metabolites, such as SCFAs or salts thereof, including at least one of acetic acid, butyric acid, valeric acid, acetate, propionate, and valerate.

In some embodiments, the culture is produced by culturing the strain in the liquid medium under anaerobic culture conditions. For example, the liquid medium is an MM01 liquid medium (including about 5 g/L of peptone, about 5 g/L of trypticase, about 10 g/L of yeast extract, about 5 g/L of beef extract, about 5 g/L of glucose, about 2 g/L of K₂HPO₄, about 2 g/L of sodium acetate, about 1 mL/L of Tween 80, about 5 mg/L of hemin, about 0.5 g/L of L-cysteine hydrochloride, about 1 µL/L of vitamin K1, and about 8 mL/L of an inorganic salt solution (including about 0.25 g/L of calcium chloride, about 1 g/L of dipotassium hydrogen phosphate, about 1 g/L of potassium dihydrogen phosphate, about 0.5 g/L of magnesium sulfate, about 10 g/L of sodium bicarbonate, and about 2 g/L of sodium chloride)). In some embodiments, the anaerobic culturing is conducted at 35°C to 42°C, such as 37°C. In some embodiments, the anaerobic culturing is conducted for 24 h to 72 h, including 36 h to 60 h, such as 48 h. In some embodiments, the anaerobic culturing is conducted at a pH of 6 to 10, such as 7.

It can be understood that the culture includes compounds and/or cellular debris of the strain, and/or metabolites secreted by the strain. The metabolites include small molecules (such as amino acids, nucleosides, and nucleotides) and macromolecules (such as polypeptides, carbohydrates, nucleic acids, proteoglycans, and lipids). The metabolites can be primary metabolites directly involved in normal cellular functions, secondary metabolites generally not required for basic cellular functions, or metabolic intermediates generated during the synthesis of primary or secondary metabolites. Thus, the similar biological functions to the strain can be achieved through different types of cultures including compounds and/or metabolites of the strain.

A third aspect of the embodiments of the present disclosure relates to a microbial agent, including one or more of the aforementioned strains or metabolites of the one or more strains or cultures of the one or more strains, for example, the strain under the accession number GDMCC No. 61980 or a metabolite of the strain or a culture of the strain.

A fourth aspect of the embodiments of the present disclosure relates to a pharmaceutical combination product, including the following independent components:
a first product including the strain described above or a metabolite of the strain described above or the culture described above or the microbial agent described above; and
a second product including a drug for combination therapy.

It can be understood that the first product includes an effective amount of the strain described above or the culture described above. The first product and the second product may be administered separately at respective doses. In this case, the first product and the second product may be administered at different time points and/or through different administration routes and/or at different administration intervals.

In some embodiments, the drug for combination therapy includes at least one of an anti-tumor drug, an anti-metabolic disease drug, an anti-inflammatory disease drug, an anti-autoimmune disease drug, an anti-infectious disease drug, and an anti-central nervous system disease drug.

In some embodiments, the anti-tumor drug may include at least one of a chemotherapeutic drug, a photosensitizer, a photothermal therapy agent, and an immunotherapeutic drug.

In some embodiments, the anti-metabolic disease drug includes drugs for treating an amino acid metabolism disorder (such as phenylketonuria, a glycine metabolism disorder, and a tyrosine metabolism disorder), an organic acid metabolism disorder (such as methylmalonic acidemia and propionic acidemia), a carbohydrate metabolism disorder (such as glycogen storage disease, galactosemia, and diabetes), a protein metabolism disorder, a lipid metabolism disorder (such as hypercholesterolemia and hyperlipidemia), a purine metabolism disorder (such as gout and Lesch-Nyhan syndrome), a pigment metabolism disorder (such as porphyria), a blood ammonia metabolism disorder (such as hyperammonemia and citrin deficiency), and a metal ion metabolism disorder (such as Wilson's disease).

With diabetes as an example, an antidiabetic drug includes at least one of insulin, metformin, pramlintide, sulfonylurea (such as glibenclamide, glipizide, and glimepiride), meglitinide (such as repaglinide and nateglinide), thiazolidinedione (such as rosiglitazone and pioglitazone), a DPP-4 inhibitor (such as sitagliptin, saxagliptin, and linagliptin), a GLP-1 receptor agonist (such as exenatide, liraglutide, and semaglutide), an SGLT2 inhibitor (such as canagliflozin, dapagliflozin, and empagliflozin), entecavir, tenofovir, lamivudine, adefovir, telbivudine, simeprevir, sofosbuvir, interferon (IFN), and ribavirin.

In some embodiments, the pharmaceutical combination product is in an infant-suitable dosage form, a child-suitable dosage form, or an adult-suitable dosage form, or the pharmaceutical combination product is in a dosage form for enteral administration or a dosage form for parenteral administration, or the pharmaceutical combination product is an oral preparation or an injection. The strain in the pharmaceutical combination product can at least partially proliferate in an intestine of a subject, and the strain may be any of a live bacterium (such as an attenuated bacterium or a lyophilized bacterium or an irradiated bacterium) and a dead bacterium.

A seventh aspect of the embodiments of the present disclosure relates to a use of the strain described above, the culture described above, the composition described above, the pharmaceutical composition described above, or the pharmaceutical combination product described above in preparation of a drug for preventing and/or treating a disease. In some embodiments, the disease includes at least one of a tumor or cancer, a metabolic disease, an inflammatory or autoimmune disease, an infectious disease, and a central nervous system disease.

The term "tumor" refers to an abnormal mass or neoplasm in the body resulting from cell division and proliferation exceeding normal levels, while the term "cancer" generally refers to a malignant tumor. In some contexts of the present disclosure, the terms "tumor" and "cancer" may be used interchangeably. The tumor or cancer typically includes at least one of a solid tumor, a soft tissue tumor, a hematopoietic tumor, a glandular tumor, and a metastatic tumor. The solid tumor can be further classified as an epithelial cell-derived tumor, a fibroblast-derived tumor, etc. based on cell origins. Further, based on different locations of primary tumor lesions, the solid tumor includes head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer. The hematopoietic tumor includes a tumor in at least one of blood, lymph, and bone marrow. The head and neck cancer includes oral cancer (such as lip cancer, tongue cancer, floor of mouth cancer, tonsil cancer, and gingival carcinoma), salivary gland cancer, nasopharyngeal cancer, oropharyngeal cancer, hypopharyngeal cancer, nasal cavity cancer, paranasal sinus cancer, laryngeal cancer, etc. The upper gastrointestinal cancer includes esophageal cancer, gastroesophageal junction cancer, gastric cancer, small intestine cancer, etc. The lower gastrointestinal cancer includes appendix cancer, colon cancer, rectal cancer, anal canal cancer, etc. The hepatobiliary system cancer includes liver cancer, intrahepatic cholangiocarcinoma, gallbladder cancer, hilar cholangiocarcinoma, distal cholangiocarcinoma, ampullary cancer, pancreatic cancer, etc. The neuroendocrine tumor includes gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, etc. The thoracic cancer includes thymic carcinoma, lung cancer (such as small cell lung cancer and non-small cell lung cancer), malignant pleural mesothelioma, etc. The soft tissue tumor includes angiosarcoma, desmoid tumor, Ewing sarcoma, fibrosarcoma (generated from fibroblasts and collagen fibers), gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, etc. The skin cancer includes Merkel cell carcinoma, cutaneous malignant melanoma, etc. The female reproductive system cancer includes vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, etc. The male reproductive system cancer includes penile cancer, prostate cancer, testicular cancer, etc. The urinary system cancer includes kidney cancer, renal pelvis cancer, ureteral cancer, bladder cancer, urethral cancer, etc. The eye cancer includes eyelid carcinoma, conjunctival carcinoma, conjunctival melanoma, uveal melanoma, retinoblastoma, lacrimal gland cancer, orbital sarcoma, ocular adnexal lymphoma, etc. The central nervous system cancer includes brain cancer (such as glioma, meningioma, medulloblastoma, medullary pituitary adenoma, primary central nervous system (CNS) lymphoma, and germ cell tumors of the central nervous system), spinal cord tumor, etc. The endocrine system cancer includes differentiated thyroid cancer, anaplastic thyroid cancer, medullary thyroid cancer, parathyroid cancer, adrenocortical carcinoma, etc. The hematopoietic tumor includes Hodgkin lymphoma, non-Hodgkin lymphoma (such as diffuse large B-cell lymphoma, follicular lymphoma, mantle cell lymphoma, Burkitt lymphoma, nodal marginal zone lymphoma, and mucosa-associated lymphoid tissue lymphoma), cutaneous lymphoma, plasma cell myeloma, leukemia, etc.

The tumor or cancer also includes a tumor or cancer caused by a bacterium or virus, such as a tumor or cancer caused by at least one bacterium or virus of hepatitis B virus, hepatitis C virus, human papillomavirus, Epstein-Barr (EB) virus, *Helicobacter pylori, and Fusobacterium nucleatum.*

The term "metabolic disease" refers to a disease caused by the accumulation or deficiency of substances such as sugars, fats, and amino acids in the human body to induce abnormal levels of metabolic substances in the human body. Metabolic diseases can generally be classified into metabolic dysfunction and hypermetabolism. In some specific embodiments of the present disclosure, the metabolic disease includes an amino acid metabolism disorder (such as phenylketonuria, a glycine metabolism disorder, and a tyrosine metabolism disorder), an organic acid metabolism disorder (such as methylmalonic acidemia and propionic acidemia), a carbohydrate metabolism disorder (such as glycogen storage disease, galactosemia, and diabetes), a lipid metabolism disorder (such as hypercholesterolemia and hyperlipidemia), a purine metabolism disorder (such as gout and Lesch-Nyhan syndrome), a pigment metabolism disorder (such as porphyria), a blood ammonia metabolism disorder (such as hyperammonemia and citrin deficiency), and a metal ion metabolism disorder (such as Wilson's disease).

In some embodiments, the inflammatory or autoimmune disease includes at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome.

In some embodiments, the infectious disease includes a disease caused by an infection with at least one of a bacterium, a virus, and a fungus. In some embodiments, the central nervous system disease includes at least one of stroke, schizophrenia, drug addiction, Alzheimer's disease, Parkinson's disease, anxiety disorder, and depression.

In some embodiments, the strain described above, the culture described above, the microbial agent described above, the composition described above, the pharmaceutical composition described above, or the pharmaceutical combination product described above prevents and/or treats a tumor through at least one of the following (a) to (l): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of a PD-1 inhibitor; (f) preventing and/or inhibiting dissemination or metastasis of a tumor cell; (g) reducing resistance to an anti-tumor drug; (h) inhibiting an activity of HDAC through at least one of SCFAs including acetic acid or acetate, propionic acid or propionate, butyric acid or butyrate, and valeric acid or valerate; (i) inhibiting an activity of HDAC and/or promoting a transcriptional activity of IFN-β; (j) modulating an immune system of a subject to play an immunomodulatory role; (k) promoting senescence or apoptosis of a tumor cell; and (l) inhibiting angiogenesis within a tumor tissue.

In some embodiments, the strain described above, the culture described above, the composition described above, the pharmaceutical composition described above, or the pharmaceutical combination product described above treats or prevents a disease (such as a tumor or cancer, a metabolic disease, an inflammatory or autoimmune disease, an infectious disease, or a central nervous system disease) by inhibiting an activity of HDAC and/or enhancing a transcriptional activity of IFN-β.

Current research results show that HDAC inhibitors exhibit significant anti-tumor effects against tumor cells at a site such as bladder, bone, breast, uterus, central nervous system, esophagus, lung, ovary, pancreas, cholangiocarcinoma, and prostate, and can induce remarkable apoptosis, proliferation inhibition, and cell cycle arrest for such tumor cells. As HDACs work like oncogenes to regulate the onset of cancers, the reduction or inhibition of activities of HDACs has been proved to enable various anti-cancer effects. Moreover, trichostatin A (TSA) can inhibit tumor angiogenesis by down-regulating the expression of tumor angiogenesis-associated genes induced by hypoxia and by directly suppressing the migration and proliferation of endothelial cells. HDAC inhibitors can also treat diabetes through a variety of mechanisms, including inhibition of Pdxl (Park et al., 2008, J Clin Invest, 118, 2316-24), enhancement of the expression of a transcription factor Ngn3 to expand the pool of endocrine progenitor cells (Haumaitre et al., 2008, Mol Cell Biol, 28, 6373-83), enhancement of the expression of insulin (Molsey et al., 2003, J Biol Chem, 278, 19660-6), etc. The HDAC inhibition is also a promising therapeutic approach for advanced diabetes-associated complications such as diabetic nephropathy and retinal ischemia (Christensen et al., 2011, Mol Med, 17 (5-6), 370-390). Therefore, the strain described above, the culture described above, the microbial agent described above, the composition described above, the pharmaceutical composition described above, or the pharmaceutical combination product described above treats or prevents a disease (such as a tumor or cancer, a metabolic disease, an inflammatory or autoimmune disease, an infectious disease, or a central nervous system disease) by inhibiting an activity of HDAC.

In some embodiments, the strain described above, the culture described above, the microbial agent described above, the composition described above, the pharmaceutical composition described above, or the pharmaceutical combination product described above treats or prevents a disease (such as a tumor or cancer, a metabolic disease, an inflammatory or autoimmune disease, an infectious disease, or a central nervous system disease) by up-regulating a pro-inflammatory factor and a chemokine to enable immune stimulation and/or by down-regulating an anti-inflammatory factor to enable immunomodulation. In some embodiments, the pro-inflammatory factor is at least one of IFN-γ, IFN-β, IL-1β, IL-6, IL-1β, MCP-1, MIG, RANTES, and TNF-α.

An eighth aspect of the embodiments of the present disclosure relates to a use of the strain described above, the culture described above, the microbial agent described above, the composition described above, the pharmaceutical composition described above, or the pharmaceutical combination product described above in prevention and/or treatment of a disease. In some embodiments, the disease includes at least one of a tumor or cancer, a metabolic disease, an inflammatory or autoimmune disease, an infectious disease, and a central nervous system disease.

In some embodiments, the strain described above, the culture described above, the microbial agent described above, the composition described above, the pharmaceutical composition described above, or the pharmaceutical combination product described above prevents and/or treats a tumor through at least one of the following (a) to (l): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of a PD-1 inhibitor; (f) preventing and/or inhibiting dissemination or metastasis of a tumor cell; (g) reducing resistance to an anti-tumor drug; (h) inhibiting an activity of HDAC through at least one of SCFAs including acetic acid or acetate, propionic acid or propionate, butyric acid or butyrate, and valeric acid or valerate; (i) inhibiting an activity of HDAC and/or promoting a transcriptional activity of IFN-β; (j) modulating an immune system of a subject to play an immunomodulatory role; (k) promoting senescence or apoptosis of a tumor cell; and (l) inhibiting angiogenesis within a tumor tissue.

In some embodiments, the strain described above, the culture described above, the microbial agent described above, the composition described above, the pharmaceutical composition described above, or the pharmaceutical combination product described above treats or prevents a disease (such as a tumor or cancer, a metabolic disease, an inflammatory or autoimmune disease, an infectious disease, or a central nervous system disease) by inhibiting an activity of HDAC and/or enhancing a transcriptional activity of IFN-β.

A ninth aspect of the embodiments of the present disclosure relates to a method for treating a disease, including administering an effective dose of the strain described above, the culture described above, the microbial agent described above, the composition described above, the pharmaceutical composition described above, or the pharmaceutical combination product described above to a subject.

In some embodiments, the disease includes at least one of a tumor or cancer, a metabolic disease, an inflammatory or autoimmune disease, an infectious disease, and a central nervous system disease. The tumor or cancer includes at least one of a solid tumor, a soft tissue tumor, a hematopoietic tumor, a glandular tumor, and a metastatic tumor. The solid tumor includes at least one of head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer. The hematopoietic tumor includes a tumor in at least one of blood, lymph, and bone marrow. The tumor or cancer includes a tumor or cancer caused by a bacterium or virus (such as at least one of hepatitis B virus, hepatitis C virus, human papillomavirus, EB virus, *Helicobacter pylori,* and *Fusobacterium nucleatum).* The metabolic disease includes a disease characterized by a metabolic disorder in at least one of an amino acid, an organic acid, a sugar, a fat, a purine, a pigment, blood ammonia, and a metal ion. The inflammatory or autoimmune disease includes at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome. The infectious disease includes a disease caused by an infection with at least one of a bacterium, a virus, and a fungus. The central nervous system disease includes at least one of stroke, schizophrenia, drug addiction, Alzheimer's disease, Parkinson's disease, anxiety disorder, and depression.

In some embodiments, preventing and/or treating a tumor is achieved through at least one of the following (a) to (l): (a) inhibiting an increase in tumor volume; (b) inhibiting an increase in tumor weight; (c) inhibiting proliferation of a tumor cell; (d) increasing a response rate of a tumor treatment; (e) enhancing therapeutic efficacy of an immunosuppressive drug, including improving therapeutic efficacy of a PD-1 inhibitor; (f) preventing and/or inhibiting dissemination or metastasis of a tumor cell; (g) reducing resistance to an anti-tumor drug; (h) inhibiting an activity of HDAC through at least one of SCFAs including acetic acid or acetate, propionic acid or propionate, butyric acid or butyrate, and valeric acid or valerate; (i) inhibiting an activity of HDAC and/or promoting a transcriptional activity of IFN-β; (j) modulating an immune system of a subject to play an immunomodulatory role; (k) promoting senescence or apoptosis of a tumor cell; and (l) inhibiting angiogenesis within a tumor tissue.

In some embodiments, the strain described above, the culture described above, the microbial agent described above, the composition described above, the pharmaceutical composition described above, or the pharmaceutical combination product described above treats or prevents a disease (such as a tumor or cancer, a metabolic disease, an inflammatory or autoimmune disease, an infectious disease, or a central nervous system disease) by inhibiting an activity of HDAC and/or enhancing a transcriptional activity of IFN-β.

In some embodiments, the strain described above, the culture described above, the microbial agent described above, the composition described above, the pharmaceutical composition described above, or the pharmaceutical combination product described above treats or prevents a disease (such as a tumor or cancer, a metabolic disease, an inflammatory or autoimmune disease, an infectious disease, or a central nervous system disease) by up-regulating a pro-inflammatory factor and a chemokine to enable immune stimulation and/or by down-regulating an anti-inflammatory factor to enable immunomodulation. In some embodiments, the pro-inflammatory factor is at least one of IFN-γ, IFN-β, IL-1β, IL-6, IL-1β, MCP-1, MIG, RANTES, and TNF-α.

In some embodiments, the strain, the culture, the microbial agent, the composition, the pharmaceutical composition, or the pharmaceutical combination product is administered at least once daily. In some embodiments, when the strain, the culture, the microbial agent, the composition, the pharmaceutical composition, or the pharmaceutical combination product is administered twice or more daily, the strain, the culture, the microbial agent, the composition, the pharmaceutical composition, or the pharmaceutical combination product is administered at a same dose. In some embodiments, when the strain, the culture, the microbial agent, the composition, the pharmaceutical composition, or the pharmaceutical combination product is administered twice or more daily, the strain, the culture, the microbial agent, the composition, the pharmaceutical composition, or the pharmaceutical combination product is administered at different doses. In some embodiments, a route for the administering includes at least one of oral administration, sublingual administration, nasal administration, rectal administration, inhalation administration, transdermal administration, intraperitoneal injection, subcutaneous injection, and intramuscular injection.

The term "subject" or "patient" refers to any mammal. The term "treating" a disease in a subject or "treating" a subject who has or is suspected of having a disease refers to administering a medication to the subject, for example, administering one or more drugs to reduce or prevent the worsening of at least one symptom of the disease. Therefore, in an embodiment, the "treating" specifically refers to delaying progression, accelerating remission, inducing remission, increasing remission, accelerating recovery, enhancing efficacy of an alternative therapy, or reducing resistance to an alternative therapy, or a combination thereof.

The term "prevention" is well known in the art. When used in the context of conditions such as local recurrence, the term "prevention" is widely recognized in the art. The term "prevention" includes the administration of a composition that reduces the occurrence of medical symptoms in a subject or delays the onset of such medical symptoms in contrast to a subject who has not received the composition. Therefore, the prevention of a cancer includes, for example, reducing the number of detectable tumors in a patient population receiving a preventive treatment in contrast to an untreated control population, and/or delaying the occurrence of detectable tumors in a treated population in contrast to an untreated control population, such as a statistically and/or clinically significant amount.

A tenth aspect of the present disclosure also relates to a preparation method of the culture described above, including the following step: inoculating the strain into a fermentation medium to produce the culture.

In some embodiments, the method further includes centrifuging the culture and collecting a supernatant resulting from the fermentation medium. In some embodiments, the method further includes concentrating the fermentation medium or the supernatant resulting from the fermentation medium through at least one of evaporation, lyophilization, dialysis, extraction, and membrane separation to produce the concentrate. In some embodiments, the method further includes subjecting the fermentation medium or the supernatant resulting from the fermentation medium to at least one of extraction and solvent extraction to produce the extract. In some embodiments, the method further includes drying any one of the fermentation medium, the supernatant resulting from the fermentation medium, the concentrate, and the extract to produce the dried product.

Meanings of some abbreviations involved in the following examples are shown in Table 1 below.

**Table 1 Definitions of some abbreviations**

| Abbreviation | Meaning/full name | Abbreviation | Meaning/full name |
|---|---|---|---|
| IND | Indole test | ESC | Esculin |
| URE | Urease | GLY | Glycerol |
| GLU | Glucose | CEL | Cellobiose |
| MAN | Mannitol | MNE | Mannose |
| LAC | Lactose | MLZ | Melezitose |
| SAC | Sucrose | RAF | Raffinose |
| MAL | Maltose | SOR | Sorbitol |
| SAL | Salicin | RHA | Rhamnose |
| XYL | Xylose | TRE | Trehalose |
| ARA | Arabinose | GEL | Gelatin |

Some media involved in the following examples are as follows:
MM01 liquid medium including the following components: 5 g/L of peptone, 5 g/L of trypticase, 10 g/L of yeast extract, 5 g/L of beef extract, 5 g/L of glucose, 2 g/L of K₂HPO₄, 2 g/L of sodium acetate, 1 mL/L of Tween 80, 5 mg/L of hemin, 0.5 g/L of L-cysteine hydrochloride, 1 µL/L of vitamin K1, and 8 mL/L of an inorganic salt solution (1 L of the inorganic salt solution includes 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride).

Solid MM01 medium including the following components: 5 g/L of peptone, 5 g/L of trypticase, 10 g/L of yeast extract, 5 g/L of beef extract, 5 g/L of glucose, 2 g/L of K₂HPO₄, 2 g/L of sodium acetate, 1 mL/L of Tween 80, 5 mg/L of hemin, 0.5 g/L of L-cysteine hydrochloride, 1 µL/L of vitamin K1, 8 mL/L of an inorganic salt solution (1 L of the inorganic salt solution includes 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride), and 15 g/L of agar.

AC liquid medium including the following components: 20 g/L of peptone, 5 g/L of glucose, 3 g/L of yeast extract, 3 g/L of beef extract, and 0.2 g/L of vitamin C. The AC liquid medium has a pH of 7.0.

Anaerobic blood agar medium (purchased from Huankai Microbial) including the following components: 10 g/L of pancreatic digest of casein, 3 g/L of pancreatic digest of heart, 1 g/L of corn starch, 5 g/L of peptic digest of meat, 5 g/L of yeast extract, 5 g/L of sodium chloride, 15 g/L of agar, 50 mL/L to 100 mL/L of sterile defibrinated sheep blood, and 1,000 mL of distilled water. The anaerobic blood agar medium has a final pH of 7.3±0.2.

TSB liquid medium including the following components: 17 g/L of pancreatic digest of casein, 3 g/L of papain digest of soybean meal, 2.5 g/L of dipotassium hydrogen phosphate, 5 g/L of sodium chloride, and 2.5 g/L of glucose. The TSB liquid medium has a pH of 7.3±0.2.

The aforementioned media can be prepared using conventional preparation processes and sterilization modes.

The above embodiments are illustrated below with reference to specific Examples.

### Example 1: Isolation and identification of a strain MNH 19801

### 1. Isolation of the strain

The strain was isolated from a fecal sample of a healthy male volunteer in Guangzhou City, Guangdong Province, China. A specific process was as follows:
2 g to 5 g of fresh feces was collected by the donor himself in a sample collection and preservation tube, thoroughly shaken for homogenization, then placed in an ice box, and delivered to the laboratory within 24 h for strain isolation.

In a biosafety cabinet, normal saline is dispensed into tubes with 9 mL per tube. Anaerobic blood agar plates for strain isolation were prepared, transferred into an anaerobic workstation (Don Whitley Scientific H35) 24 h in advance, and labeled for sample information, medium types, isolation date, etc.

The fresh fecal sample was placed in the anaerobic workstation, and vortexed by a vortex shaker for 1 min to enable thorough mixing. 1 mL of a resulting sample was taken and added to 9 mL of the normal saline, and thorough mixing was conducted to produce a 10⁻¹ dilution. Then, the 10⁻¹ dilution was serially diluted to produce a 10⁻⁶ dilution for later use.

The 10⁻⁶ dilution was added dropwise on isolation media (anaerobic blood agar plates, Columbia blood agar plates, and chocolate agar plates) at 100 µL/plate, and spread evenly. After a surface of a plate was dried, the plate was inverted and incubated at 37°C for 3 d to 5 d.

A growth status of a strain in an isolation medium was observed, and single colonies were picked using a sterilized toothpick for strain purification. A purified strain was cultured anaerobically at 37°C.

The purified strain MNH 19801 cultured was prepared into a 20% glycerol/water-based bacterial suspension and stored at -86°C.

### 2. Identification of the strain

### 2.1 Morphological characteristics

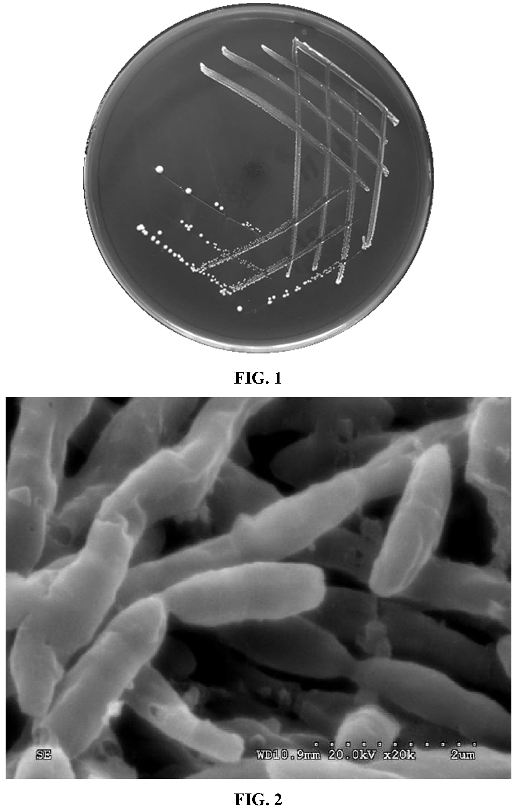

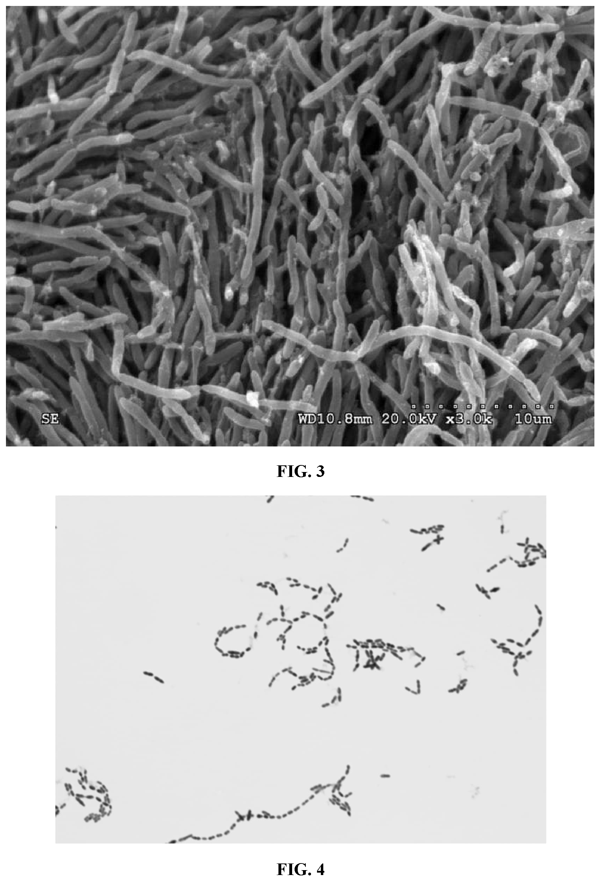

The strain MNH 19801 was inoculated on an anaerobic blood agar plate, cultured anaerobically at 37°C for 48 h, and then observed. An observation result was shown in FIG. 1. As shown in this figure, visible colonies were generated on the anaerobic blood agar plate. These colonies were circular with regular edges, white, and opaque, and had a diameter of approximately 2 mm. No secretions were produced around these colonies. As shown in FIG. 2 to FIG. 5, morphological observation results under a microscope revealed that the strain MNH 19801 did not have spores and flagella and was non-motile and Gram-positive.

### 2.2 Physiological and biochemical characteristics

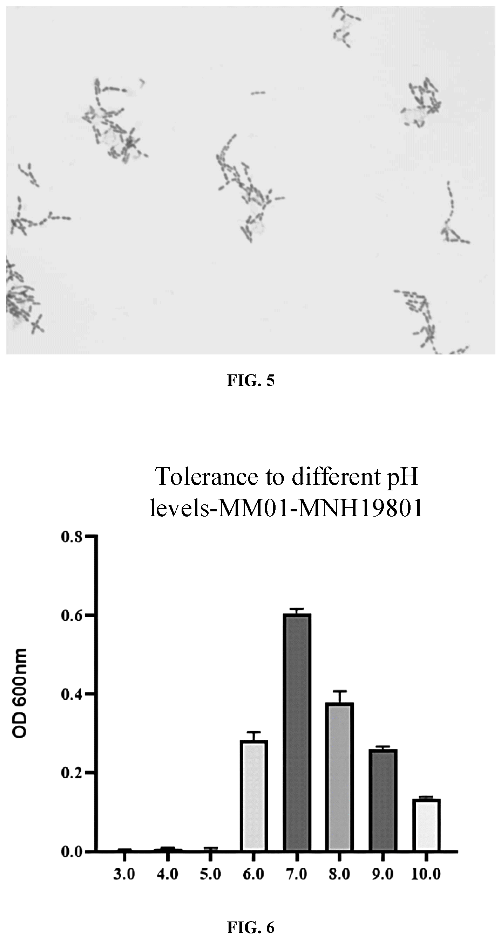

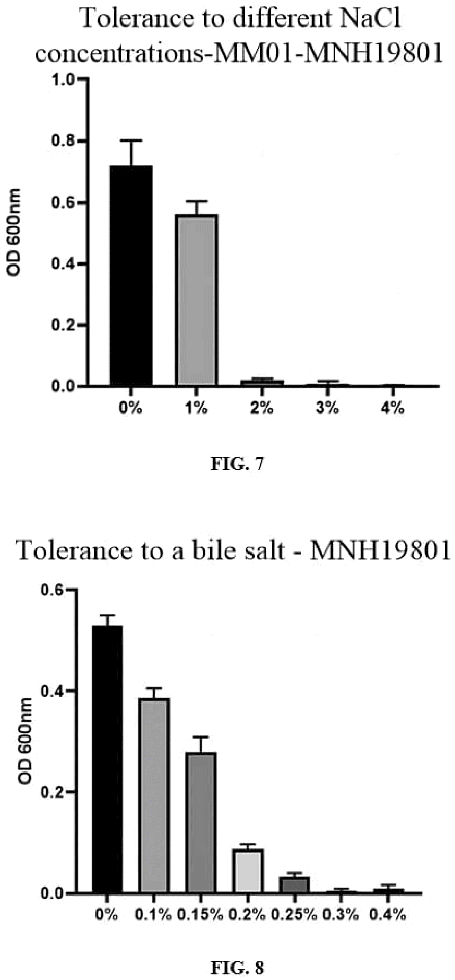

A growth temperature range for the strain MNH 19801 was 30°C to 42°C, and an optimal growth temperature for this strain was 37°C. As shown in FIG. 6, this strain could grow in a pH range of 6.0 to 10.0, with an optimal growth pH of 7.0. As shown in FIG. 7 to FIG. 8, the strain MNH 19801 could not grow at a NaCl concentration of more than 2%. This strain can survive and grow in a bile salt concentration range of 0% to 0.25%, but the growth of this strain trended to be weakened with the increase in bile salt concentration. The strain MNH 19801 could not grow under aerobic conditions, but grew well under anaerobic conditions. Thus, the strain MNH 19801 belongs to obligate anaerobic bacteria.

### API 20A test

The API 20A test was conducted using an API 20A test strip (BioMérieux) according to instructions. The strain was cultured anaerobically at 37°C. Test results were shown in Table 2.

**Table 2 API 20A test results for the strain MNH 19801**

| Test item | Test result | Test item | Test result |
|---|---|---|---|
| IND | Negative | ESC | Negative |
| URE | Negative | GLY | No acid production |
| GLU | No acid production | CEL | No acid production |
| MAN | No acid production | MNE | Acid production |
| LAC | No acid production | MLZ | No acid production |
| SAC | No acid production | RAF | Acid production |
| MAL | No acid production | SOR | No acid production |
| SAL | Acid production | RHA | Acid production |
| XYL | Acid production | TRE | No acid production |
| ARA | No acid production | GEL | Negative |

It can be seen from this table that the strain MNH 19801 can utilize mannitol, raffinose, xylose, salicin, and rhamnose as substrates for metabolism to produce acids, but cannot utilize other types of substrates.

### Antibiotic sensitivity test

The strain MNH 19801 was subjected to the antibiotic sensitivity test by the disk diffusion method, and test results were shown in Table 3.

**Table 3 Antibiotic sensitivity test results for the strain MNH 19801**

| Antibiotic | Inhibition zone diameter (mm) |
|---|---|
| Gentamicin (GEN) | 10.74 |
| Erythromycin (ERM) | 111.41 |
| Chloramphenicol (CLM) | 30.39 |
| Tetracycline (TET) | 12.88 |
| Penicillin (PEN) | 18.79 |
| Ciprofloxacin (CFX) | 0 |
| Trimethoprim/Sulfamethoxazole (T/S) | 0 |
| Ampicillin (AMP) | 11.17 |
| Lincomycin (LIN) | 0 |
| Ceftriaxone (CTR) | 33.18 |

According to the results in Table 3, the strain MNH 19801 is sensitive to antibiotics including gentamicin, erythromycin, chloramphenicol, tetracycline, penicillin, ampicillin, and ceftriaxone, and is resistant to ciprofloxacin, trimethoprim/sulfamethoxazole, and lincomycin.

### 2.3 16S rRNA

A fresh culture of the strain MNH 19801 was taken. Genomic DNA was extracted from the sample using the sodium dodecyl sulfate (SDS) method. The purity and integrity of the DNA were then detected by agarose gel electrophoresis.

Genomic DNA was extracted from the sample using the SDS method. The purity and integrity of the DNA were then detected by agarose gel electrophoresis. The DNA was quantified by Qubit. The extracted genomic DNA was used for library construction and sequencing on Illumina and PacBio platforms.

For library construction and sequencing on the Illumina platform, a DNA sample that passed electrophoresis quality control was randomly fragmented into fragments of approximately 350 bp using a Covaris ultrasonic disruptor. The DNA fragments were used to complete the entire library preparation through steps such as end repair, A-tailing, adapter ligation, purification, and PCR amplification with the NEBNext^{®}Ultra^{™} DNA Library Prep Kit for Illumina (NEB, USA). After library construction, initial quantification was performed using Qubit 2.0, and a library was diluted to 2 ng/µL. An insert size of the library was then detected using Agilent 2100. Once the insert size met an expectation, an effective concentration of the library was accurately quantified by the Q-PCR method to ensure a quality of the library. After the library passed a quality inspection, PE150 sequencing was performed using the Illumina NovaSeq platform.

For library construction and sequencing on the PacBio platform, an SMRT Bell library was constructed with the SMRT bell TM Template kit (version 2.0). A DNA sample that passed electrophoresis quality control was fragmented into target fragments with a size required for library construction using Covaris g-TUBE. DNA damage repair and end repair were conducted, and a hairpin adapter was ligated to two ends of each DNA fragment using DNA ligase. DNA fragments were purified using AMpure PB beads, and fragments of a specific size were selected using Blue Pippin. A concentration of the SMRT Bell library was adjusted with AMpure PB beads. DNA damage repair was conducted. The SMRT Bell library was purified using AMpure PB beads. A constructed library was quantified for a concentration by Qubit, and an insert size was detected using Agilent 2100. Finally, a qualified library was sequenced on the PacBio Sequel II platform.

1.006 Gbp of raw data was acquired from the sequencing on the PacBio platform, with an average sequence length of 82,146 bp. 3.25 Gbp of raw data was acquired from the sequencing on the Illumina platform, with a Q20 proportion of 96.19%.

Adapter removal was first performed for the raw PacBio sequencing data to obtain high-quality data for subsequent analysis. Based on the high-quality data, genome assembly was performed with Canu software to obtain a preliminary assembly result reflecting the basic genomic characteristics of the sample. The preliminary assembly result was subjected to error correction with pbmm2 software based on third-generation sequencing data, and then corrected using Pilon software based on Ilumina sequencing data to obtain a final assembly result. After the assembled genome was obtained, a full-length 16S sequence was extracted from the assembled genome using CheckM software. A 16S sequence of 1,381 bp was acquired from Sanger sequencing, and a 16S sequence of 1,540 bp was extracted from the assembled genome. The two sequences were aligned using blastn. Alignment results were as follows: identity: 100.0% and coverage: 100.0%. As a result, the 16S sequence of 1,540 bp extracted from the assembled genome was selected for species identification.

A sequence of a 16S rRNA gene of the strain MNH 19801 was as follows:

AACTTTATTAGAGAGTTTGATCCTGGCTCAGGATGAACGCTGGCGGCGTGCTT
AACACATGCAAGTCGAACGAAGCGCTTGAGAAGATTTCTTCGGAATGAAACTTAAGT
GACTGAGTGGCGGACGGGTGAGTAACGCGTGGGTAACCTGCCTCATACAGGGGGATA
ACAGTCAGAAATGACTGCTAATACCGCATAAGCGCACAGTACCGCATGGTACGGTGT
GAAAAACTCCGGTGGTATGGGATGGACCCGCGTCTGATTAGCTAGTTGGTGGGGTAA
CGGCCTACCAAGGCGACGATCAGTAGCCGGCCTGAGAGGGCGACCGGCCACATTGG
GACTGAGACACGGCCCAAACTCCTACGGGAGGCAGCAGTGGGGAATATTGCACAAT
GGGGGAAACCCTGATGCAGCGACGCCGCGTGAGCGAAGAAGTATTTCGGTATGTAAA
GCTCTATCAGCAGGGAAGAAAATGACGGTACCTGACTAAGAAGCCCCGGCTAACTAC
GTGCCAGCAGCCGCGGTAATACGTAGGGGGCAAGCGTTATCCGGATTTACTGGGTGTA
AAGGGAGCGTAGACGGACTGGCAAGTCTGATGTGAAAACCCGGGGCTCAACCCCGG
GACTGCATTGGAAACTGTCAGTCTAGAGTGTCGGAGGGGTAAGTGGAATTCCTAGTG
TAGCGGTGAAATGCGTAGATATTAGGAGGAACACCAGTGGCGAAGGCGGCTTACTGG
ACGATAACTGACGTTGAGGCTCGAAAGCGTGGGGAGCAAACAGGATTAGATACCCTG
GTAGTCCACGCCGTAAACGATGAATACTAGGTGTCGGGAAGCAAAGCTTTTCGGTGC
CGCAGCAAACGCAATAAGTATTCCACCTGGGGAGTACGTTCGCAAGAATGAAACTCA

AAGGAATTGACGGGGACCCGCACAAGCGGTGGAGCATGTGGTTTAATTCGAAGCAA
CGCGAAGAACCTTACCAAGTCTTGACATCGATCTGCCCGGACTGTAACGAGTCCTTTC
CCTTCGGGGACAGAGAAGACAGGTGGTGCATGGTTGTCGTCAGCTCGTGTCGTGAG
ATGTTGGGTTAAGTCCCGCAACGAGCGCAACCCTTATCCTTAGTAGCCAGCAGGTAA
AGCTGGGCACTCTGGGGAGACTGCCAGGGATAACCTGGAGGAAGGTGGGGATGACG
TCAAATCATCATGCCCCTTATGATTTGGGCTACACACGTGCTACAATGGCGTAAACAA
AGGGAAGCAGCCCCGCGAGGGTGAGCAAATCTCAAAAATAACGTCCCAGTTCGGAT
TGTAGTCTGCAACTCGACTACATGAAGCTGGAATCGCTAGTAATCGCAGGTCAGCATA
CTGCGGTGAATACGTTCCCGGGTCTTGTACACACCGCCCGTCACACCATGGGAGTCG
GATATGCCCGAAGCCAGTGACCCAACCTTAGGGAGGGAGCTGTCGAAGGTGGAGCC
GATAACTGGGGTGAAGTCGTAACAAGGTAGCCGTATCGGAAGGTGCGGCTGGATCAC
CTCCTTTCT(SEQ ID NO: 1).

The obtained sequence was aligned with data in GenBank using BLAST. According to alignment results, the strain MNH 19801 belonged to the family *Lachnospiraceae.* Strains with high similarities to MNH 19801 were *Blautia brookingsii* (96.031%), *Blautia faecicola* (95.366%), *Robinsoniella peoriensis* (93.91%), and *Oliverpabstia intestinalis(95.427%).* The strain MNH 19801 demonstrated a 16S rRNA gene sequence similarity of lower than 95% to other strains in the family *Lachnospiraceae.* The low 16S rRNA gene sequence similarity (less than 96.031%) indicated that the strain MNH 19801 may belong to a novel genus in the family *Lachnospiraceae* or may be a novel species in the family *Lachnospiraceae.*

The 16S rRNA gene sequence of MNH 19801 was compared with 16S rRNA gene sequences of relevant strains in the family *Lachnospiraceae* retrieved from databases such as GenBank to construct a phylogenetic tree. It can be seen from the phylogenetic tree that the strain MNH 19801 and the strain *Blautia faecicola* KGMB01111^{T} (MK424331, the superscript ^{T} denotes a type strain) of the genus *Blautia* together constitute a separate evolutionary branch (Bootstrap value: 86).

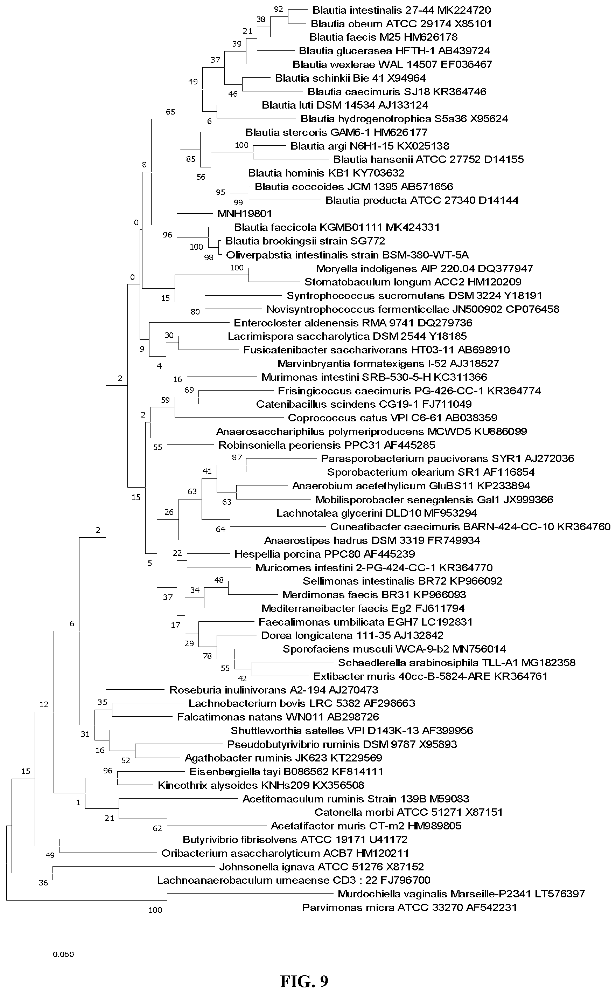

MNH 19801 and type strains from the NCBI database that had a high 16S rRNA gene sequence similarity to MNH 19801 were subjected to a multiple sequence alignment, and a phylogenetic tree was then constructed using software MEGA 5 by the maximum likelihood method. The phylogenetic tree was shown in FIG. 9, where only nodes with Bootstrap values greater than 50% were displayed. It can be seen from this phylogenetic tree that the strain MNH 19801 constitutes two distinct evolutionary branches (Bootstrap values: 96) with the strain *Blautia faecicola* KGMB01111T (MK424331) of the genus *Blautia* and the *Blautia brookings* ii strain SG772 of the genus *Blautia,* respectively.

### 2.4 Genome analysis

Based on the genome data obtained from the assembly above, genomic gene prediction analysis was performed using prokaryotic genome annotation software prokka (version: 1.14.6). 3,292 coding sequences (CDSs) were predicted in total, with an average CDS length of 930 bp. The type strain *Blautia faecicola* showed the highest genome similarity to MNH 19801, with an ANI value of 80.29% and a gene coverage of 22.04%. Therefore, MNH 19801 could be determined as a novel genus in the family *Lachnospiraceae.*

Potential antibiotic resistance genes in the genome of MNH 19801 were analyzed using the Resistance Gene Identifier (RGI) (version: 4.2.2), with the Comprehensive Antibiotic Resistance Database (CARD) (version: 3.0.0, https://card.mcmaster.ca/analyze/rgi) as an antibiotic resistance gene database. No antibiotic resistance genes were identified.

Potential virulence factors and related genes in the genome of MNH 19801 were analyzed using NCBI blastp (version: 2.10.1+) in contrast to the Virulence Factor Database (VFDB) (http://www.mgc.ac.cn/cgi-bin/VFs/v5/main.cgi, updated on September 19, 2019). Detailed alignment results were shown in Table 4.

**Table 4 Potential virulence genes of MNH19801**

| Strain gene | VFDB gene | Gene name | Identity (%) |
|---|---|---|---|
| gene_00810 | VFG000077 | clpP | 70.984 |
| gene_00839 | VFG013286 | galE | 64.583 |
| gene_01307 | VFG001967 | glf | 62.707 |

Potential secondary metabolite-synthesizing gene clusters in the genome of MNH 19801 were analyzed using antiSMASH5 (version: 5.1.1). Detailed alignment results were shown in Table 5.

**Table 5 Potential secondary metabolite-synthesizing gene clusters of MNH19801**

| Gene cluster region | Type | From | To | The most similar known gene cluster | Similarity |
|---|---|---|---|---|---|
| Region 1.1 | cyclic-lactone-autoinducer | 28457 | 49067 | - | - |
| Region 1.2 | thioamide-NRP | 613306 | 654826 | - | - |
| Region 2.1 | ranthipeptide | 5567 | 27200 | - | - |
| Region 2.2 | cyclic-lactone-autoinducer | 113299 | 133587 | - | - |
| Region 2.3 | cyclic-lactone-autoinducer,thio peptide | 290405 | 324957 | - | - |

Potential primary metabolite-synthesizing gene clusters in the genome of MNH 19801 were analyzed using gutSMASH5 (version: 1.0.0). Detailed alignment results were shown in Table 6.

**Table 6 Potential primary metabolite-synthesizing gene clusters of MNH19801**

| Gene cluster region | Type | From | to | The most similar known gene cluster | Abbreviation | Similarity |
|---|---|---|---|---|---|---|
| Region 1.1 | Putrescine2spermidineArginine2putrescine | 581750 | 608157 | Arginine2putrescine R. gnavus | PUTR | 100 % |
| Region 1.2 | pdu | 1040337 | 1087023 | Propanediol degradation S. enterica | PDU | 50% |
| Region 2.1 | Pyruvate2acetate-formate,PFOR_II_pathway | 99932 | 140546 | Pyruvate to acetate-formate E. coli | PFL_a cetate | 100 % |
| Region 3.1 | succinate2propionate,Rnf_ complex | 129873 | 177538 | Rnf complex C. sporogenes | RNF | 100 % |
| Region 10.1 | TPP_AA_metabolism,Othe rs_HGD_unassigned | 1 | 17241 | - | - | - |

A butyric acid-producing capacity of this strain was evaluated with the butyric acid synthesis pathway-related genes in the art (Vital M, Howe C, Tiedje M. Revealing the Bacterial Butyrate Synthesis Pathways by Analyzing (Meta) genomic Data [J]. Mbio, 2014, 5 (2): 1-11.) as a reference database. A genomic sequence of this strain was aligned with the reference database using NCBI blastp (version: 2.10.1+). Detailed alignment results were shown in Table 7. The completeness of a butyric acid synthesis pathway was calculated. According to a calculation, the completeness of the butyric acid synthesis pathway in this strain was 10%.

**Table 7 Potential butyric acid-producing genes in MNH19801**

| Strain gene | Butyric acid synthesis pathway | Gene name | Identity (%) |
|---|---|---|---|
| gene_01750 | Butyrat_P3 | HgdB | 73.315 |
| gene_02233 | Butyrat_P3 | HgdB | 83.77 |

### Fatty acid composition analysis

The strain MNH 19801 was inoculated on TSAplates and anaerobically cultured at 37°C for 48 h. Bacterial cells were then collected and subjected to fatty acid extraction and methylation. The strain MNH 19801 was subjected to fatty acid composition analysis with a fully automated microbial identification system from MIDI Inc., USA (Microbial ID, Inc., Newark, Del) (Kroppenstedt, 1985; Meier et al., 1993).

A fatty acid composition of the experimental strain MNH 19801 was shown in Table 8. The major fatty acids (higher than 10%) produced by MNH 19801 were hexadecanoic acid (C16:0, 28.92%) and isohexadecanoic acid (C16:0 DMA, 17.02%). Details for other fatty acid types and contents thereof were shown in the table below.

**Table 8 Fatty acid composition of the strain MNH 19801**

| RT | Response | Ar/H | RFac | ECL | Peak | Percen | Comment1 | Comment2 |
|---|---|---|---|---|---|---|---|---|
| 1.626 | 9.413E+ | 0.045 | ---- | 6.976 | SOLVEN | ---- | < min rt | ---- |
| 2.290 | 347 | 0.037 | ---- | 8.248 | ---- | ---- | < min rt | ---- |
| 4.870 | 555 | 0.031 | 1.054 | 12.00 | 12:0 | 0.58 | ECL | Reference |
| 5.965 | 494 | 0.026 | 1.020 | 12.93 | Sum In | 0.50 | ECL | 13:1 CIS 12 |
| 7.173 | 579 | 0.044 | ---- | 13.80 | ---- | ---- | ---- | ---- |
| 7.439 | 7118 | 0.039 | 0.989 | 13.99 | 14:0 | 7.04 | ECL | Reference |
| 8.179 | 1571 | 0.038 | 0.978 | 14.47 | 14:0 | 1.54 | ECL | Reference |
| 8.925 | 3572 | 0.041 | 0.968 | 14.95 | 16:0 | 3.45 | ECL | Reference |
| 9.002 | 933 | 0.034 | 0.967 | 15.00 | 15:0 | 0.90 | ECL | Reference |
| 9.806 | 445 | 0.038 | 0.958 | 15.47 | Sum In | 0.43 | ECL | 15:0 DMA |
| 10.30 | 392 | 0.032 | 0.953 | 15.77 | 16:1 CIS 7 | 0.37 | ECL | ---- |
| 10.37 | 1317 | 0.038 | 0.953 | 15.81 | 16:1 CIS 9 | 1.25 | ECL | ---- |
| 10.68 | 30465 | 0.044 | 0.950 | 15.99 | 16:0 | 28.92 | ECL | Reference |
| 11.18 | 735 | 0.037 | 0.946 | 16.28 | 16:1 CIS 9 | 0.69 | ECL | ---- |
| 11.50 | 18053 | 0.043 | 0.944 | 16.47 | 16:0 | 17.02 | ECL | Reference |
| 11.93 | 1201 | 0.038 | 0.940 | 16.71 | 17:0 | 1.13 | ECL | ---- |
| 12.01 | 877 | 0.036 | 0.940 | 16.76 | Sum In | 0.82 | ECL | 17:1 CIS 8 |
| 12.08 | 932 | 0.038 | 0.940 | 16.80 | Sum In | 0.88 | ECL | 17:2 FAME |
| 12.38 | 1400 | 0.059 | 0.938 | 16.97 | 18:0 | 1.31 | ECL | ---- |
| 13.25 | 530 | 0.038 | 0.933 | 17.46 | 17:0 | 0.49 | ECL | Reference - |
| 13.70 | 3519 | 0.044 | 0.930 | 17.72 | 18:2 CIS | 3.27 | ECL | ---- |
| 13.78 | 3295 | 0.045 | 0.930 | 17.77 | 18:1 CIS 9 | 3.06 | ECL | ---- |
| 13.88 | 3751 | 0.049 | 0.930 | 17.82 | Sum In | 3.48 | ECL | 18:1c11/t9/t |
| 14.19 | 5210 | 0.046 | 0.928 | 18.00 | 18:0 | 4.83 | ECL | Reference - |
| 14.50 | 4120 | 0.046 | 0.927 | 18.17 | Sum In | 3.82 | ECL | 18:2 DMA |
| 14.59 | 5240 | 0.047 | 0.926 | 18.22 | 18:1 CIS 9 | 4.85 | ECL | ---- |
| 14.69 | 4744 | 0.048 | 0.926 | 18.28 | 18:1 CIS | 4.39 | ECL | ---- |
| 15.01 | 5368 | 0.047 | 0.925 | 18.46 | 18:0 | 4.96 | ECL | ---- |
| ---- | 494 | ---- | ---- | ---- | Summed | 0.50 | 13:1 CIS 12 | 14:0 ALDE |
| ---- | ---- | ---- | ---- | ---- | ---- | ---- | 11:1 2OH | ---- |
| ---- | 445 | ---- | ---- | ---- | Summed | 0.43 | 15:0 DMA | 14:0 3OH |
| ---- | 877 | ---- | ---- | ---- | Summed | 0.82 | 17:2 FAME | 17:1 CIS 8 |
| ---- | 932 | ---- | ---- | ---- | Summed | 0.88 | 17:1 CIS 9 | 17:2 FAME |
| ---- | 3751 | ---- | ---- | ---- | Summed | 3.48 | 18:1c11/t9/t | UN 17.834 |
| ---- | 4120 | ---- | ---- | ---- | Summed | 3.82 | 17:0 ISO | 18:2 DMA |

In the table above, "----" indicates irrelevant data.

Based on data including morphological characteristics, microscopic features, physiological and biochemical properties, and 16S rRNA gene sequence and genome information of the strain MNH 19801, it is determined that the strain MNH 19801 belongs to a novel genus in the family *Lachnospiraceae.*

The strain MNH 19801 was deposited in the Guangdong Microbial Culture Collection Center (GDMCC) under an accession number GDMCC No. 61980 on October 27, 2021, with a taxonomic designation of *Lachnospiraceae* sp. MNH19801. The Guangdong Microbial Culture Collection Center (GDMCC) is located at the Guangdong Institute of Microbiology, Guangdong Academy of Sciences on No. 59 Building, No. 100, Xianliezhong Road, Guangzhou. It has been confirmed by the Guangdong Microbial Culture Collection Center (GDMCC) that the deposited strain remains viable.

### Example 2: Determination of SCFAs

Preparation of bacterial cells: The strain MNH 19801 was inoculated into a TSB liquid medium and cultured anaerobically at 37°C for 48 h. The bacterial cells were collected through centrifugation and stored at -86°C for later use.

Preparation of standard solutions: Acetic acid, propionic acid, butyric acid, isobutyric acid, valeric acid, isovaleric acid, and hexanoic acid standards were weighed and prepared with ethyl acetate into eight mixed standard solutions with concentrations of 0.1 µg/mL, 0.5 µg/mL, 1 µg/mL, 5 µg/mL, 10 µg/mL, 20 µg/mL, 50 µg/mL, and 100 µg/mL, respectively. 600 µL of a standard solution was taken, 25 µL of 4-methylvaleric acid was added at a final concentration of 500 µM as an internal standard, and thorough mixing was conducted. A resulting mixture was transferred into a sample vial, and analyzed by gas chromatography-mass spectrometry (GC-MS) with an injection volume of 1 µL and a split ratio of 10:1 (split injection).

Metabolite extraction: A sample was thawed on ice. 80 mg of a thawed sample was added to a 2 mL glass centrifuge tube, resuspended with 900 µL of 0.5% phosphoric acid, shaken for 2 min, and centrifuged at 14,000 g for 10 min. 800 µL of a resulting supernatant was vortexed, and an equal amount of ethyl acetate was added for extraction. A resulting mixed solution was vortexed for 2 min, and then centrifuged at 14,000 g for 10 min. 600 µL of a resulting organic phase in an upper layer was collected, 4-methylvaleric acid was added at a final concentration of 500 µM as an internal standard, and thorough mixing was conducted. A resulting test sample was transferred into a sample vial, and analyzed by GC-MS with an injection volume of 1 µL and a split ratio of 10:1 (split injection).

Sample analysis: A sample was subjected to separation using an Agilent DB-WAX capillary column (30 m × 0.25 mm ID × 0.25 µm) gas chromatography system. Temperature programming: An initial temperature was 90°C. A temperature was raised at 10°C/min to 120°C, then at 5°C/min to 150°C, and finally at 25°C/min to 250°C, and kept at 250°C for 2 min. A carrier gas was helium at a flow rate of 1.0 mL/min.

Mass spectrometry (MS) analysis was conducted using an Agilent 7890A/5975C GC-MS system. An inlet temperature was 250°C, an ion source temperature was 230°C, a transfer line temperature was 250°C, and a quadrupole temperature was 150°C. An electron ionization (EI) source and full scan and selected ion monitoring (SIM) modes were adopted. An electron energy was 70 eV.

A chromatographic peak area and a retention time were extracted using MSD ChemStation software. A standard curve was plotted. A content of SCFAs in the sample was calculated.

**Table 9 SCFA yield results of the strain MNH 19801**

| SCFA type | Yield (µg/g) |
|---|---|
| Acetic acid | 1429.94 |
| Propionic acid | 3.78 |
| Isobutyric acid | 0.08 |
| Butyric acid | 9.04 |
| Isovaleric acid | 1.26 |
| Valeric acid | 0.07 |
| Hexanoic acid | 0.34 |

According to the results, the strain MNH 19801 can synthesize acetic acid in a large quantity and can synthesize propionic acid, isovaleric acid, butyric acid, isobutyric acid, hexanoic acid, and valeric acid in small quantities during a growth process.

### Example 3: Preparation method for bacterial cells of MNH 19801

Single colonies of MNH 19801 were picked with a sterile toothpick, inoculated into 10 mL of an AC liquid medium, and cultured anaerobically at 37°C for 1 d to produce a first bacterial suspension. Then, 1 mL of the first bacterial suspension was taken and identified by MS. If an identification result was positive, 2 mL of the first bacterial suspension was transferred into 80 mL of an AC liquid medium and cultured in an anaerobic workstation at 37°C for 24 h to produce a second bacterial suspension. 30 mL of the second bacterial suspension was transferred into 400 mL of an AC liquid medium and cultured in an anaerobic workstation at 37°C for 24 h to produce a third bacterial suspension. 1 mL of the third bacterial suspension was collected and identified by MS. If an identification result was positive, the third bacterial suspension was totally transferred into a 1 L centrifuge bottle and centrifuged for 30 min at 6,000 rpm and 4°C to produce a pellet. The pellet was resuspended in a mixture of an AC liquid medium and sterile glycerol (4:1) to produce an MNH 19801 bacteria-glycerol mixed solution.

0.1 mL of the MNH 19801 bacteria-glycerol mixed solution was added to 0.9 mL of normal saline, and serially diluted to a concentration of 10⁻⁸. Then, 100 µL of each of dilutions at concentrations of 10⁻⁶, 10⁻⁷, and 10⁻⁸ was added to an anaerobic blood agar plate, glass beads were added, and thorough mixing was conducted. A viable bacterial concentration (CFU) was determined by the plate count method.

The prepared bacterial dilutions were each dispensed into sterile cryovials with 0.2 mL per cryovial and 10 cryovials for each concentration, and frozen at -80°C. After complete freezing (24 h), a cryovial of frozen bacteria was taken out, thawed to room temperature, and tested for CFU by the dilution plating method for later use.

### Example 4: Regulation of the immune activity of macrophages by the strain MNH 19801

The strain MNH 19801 was co-cultured with macrophages (purchased from Wuhan Pricella Biotechnology Co., Ltd.) for 24 h. A culture supernatant was collected and tested for a concentration of a cytokine IL-1β by enzyme-linked immunosorbent assay (ELISA). A specific process could be seen in Example 1 of CN112618576A.

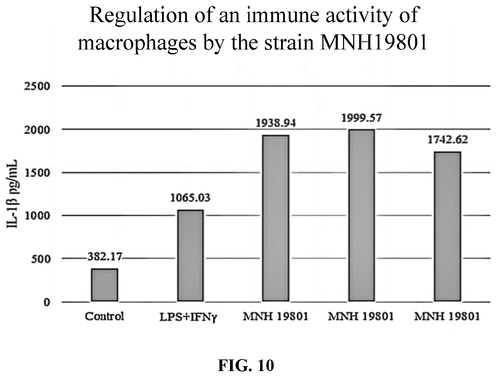

Results were shown in FIG. 10. After the macrophages were co-cultured with the strain MNH 19801, the macrophages exhibited the distinct characteristic of differentiation toward M1 macrophages, with a significant increase in IL-1β secretion.

After the strain MNH 19801 was co-cultured with the macrophages for 24 h, a culture supernatant was collected and tested for concentrations of pro-inflammatory cytokines IL-6, TNF-α, RANTES (CCL5), MIG (CXCL-9), MCP-1 (CCL2), IL-2, IL-1β, IL-10, IFN-γ, IP-10, and IL-12/IL-23P40 by cytometric bead array (CBA) assay. Results showed that, after the macrophages were co-cultured with MNH20237, the macrophages exhibited the significant characteristic of differentiation toward M1 macrophages. Specifically, the secretion of any of IL-6, RANTES, MIG, MCP-1, IL-1β, IP-10, and IL-12/IL-23P40 was very significantly enhanced.

### Example 5: Regulation of the immune activity of PBMCs by the strain MNH 19801

The strain MNH 19801 was co-cultured with primary PBMCs (purchased from TPCS, Batch No. A19Z289100) for 24 h. A culture supernatant was collected and tested for concentrations of cytokines IL-1β and IFN-γ by ELISA. A specific process could be seen in Example 2 of CN112618576A.

After the strain MNH 19801 was co-cultured with primary PBMCs for 24 h, a culture supernatant was collected and tested for concentrations of cytokines TNFα, RANTES (CCL5), MIG (CXCL-9), IL-12/IL-23P40, IL-1β, IL-6, IL-8, MCP-1 (CCL2), and IP-10 by CBA assay.

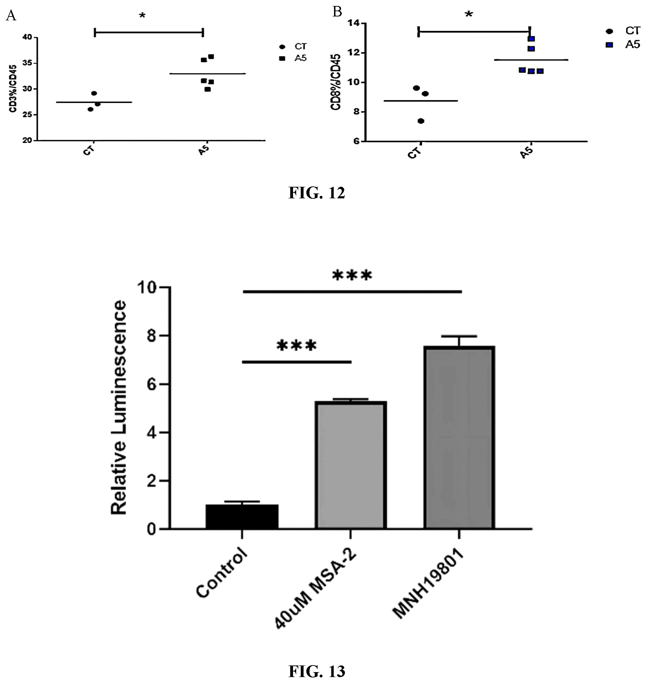

Results were shown in FIG. 13. MNH19801 could induce the increased expression of inflammatory cytokines TNF-α, IL-1β, IL-6, and MCP-1.

### Example 6: Influence of the strain MNH19801 on the expression of IFN-β

To verify whether MNH19801 could promote the expression of IFN-β, THP-1 cells carrying an IFN-β promoter reporter (a cell line established by MoonBiotech) were used to evaluate the influence of MNH19801 on a transcriptional activity of IFN-β.

Steps for constructing the THP-1 cells carrying the IFN-β promoter reporter (THP-1-IFNβ-promoter reporter) were as follows: A reporter gene was inserted into a vector, a vector with the reporter gene inserted was used to infect cells, and screening was conducted to obtain the cell line expressing the reporter gene (specifically, Du H, Xu T, Cui M. cGAS-STING signaling in cancer immunity and immunotherapy. Biomed Pharmacother. 2021;133:110972.; Jiang M, Chen P, Wang L, et al. cGAS-STING, an important pathway in cancer immunotherapy. J Hematol Oncol. 2020;13(1):81.; Lam KC, Araya RE, Huang A, et al. Microbiota triggers STING-type I IFN-dependent monocyte reprogramming of the tumor microenvironment. Cell. 2021;184(21):5338-5356.e21.).

Preparation of an MNH19801 culture supernatant: The strain MNH19801 was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

Samples to be tested included:
1) Control group: An MM01 liquid medium was diluted with phosphate buffered saline (PBS) to a volume percentage of 10%.
2) Positive control MSA-2 group: MSA-2 (Sting agonist, TargetMol Cat No. T8798) was diluted with PBS to a final concentration of 40 µM.
3) MNH19801 group: The MNH19801 culture supernatant was diluted with PBS to a volume percentage of 10%.

THP-1-IFNβ-promoter reporter cells were inoculated into a 96-well plate at 1 × 10⁵ cells/well. The cells were treated according to the established groups. The cells were further cultured for 24 h. Centrifugation was conducted at 300 g for 5 min. A resulting culture supernatant was discarded, 50 µL of a 1 × Luciferase detection reagent was added, and a reaction was conducted for 1 min. A luminescence value was measured by a microplate reader. Relative luminescence values were normalized to the control group to evaluate the influence of MNH19801 on the transcriptional activity of IFN-β.

Results were shown in FIG. 12. MNH19801 could significantly enhance the transcriptional activity of IFN-β, with a stronger enhancing effect than the positive control MSA-2.

IFNs are a class of cytokines secreted by host cells that can regulate immune responses. Viruses, bacterial endotoxins, and synthetic double-stranded RNAs can stimulate the production of IFNs. Macrophages, lymphocytes, and somatic cells in the human body can all produce IFNs. IFN-β is type I IFN. IFN-β can enhance the activities of natural killer (NK) cells, macrophages, and T lymphocytes to exert anti-viral, anti-tumor, and immunomodulatory effects. Therefore, the results of this example indicate that MNH19801 can play immunomodulatory, anti-viral, and anti-tumor roles by promoting the transcriptional activity of IFN-β.

### Example 7: Inhibitory effect of the strain MNH19801 on the activity of HDAC

To verify whether MNH19801 could inhibit the activity of HDAC, an HDAC Inhibitor Drug Screening Kit (Fluorometric) purchased from Abcam was used to detect the *in vitro* inhibitory effect on the activity of HDAC in this example.

Preparation of an MNH19801 culture supernatant: The strain MNH19801 was inoculated into a liquid medium and cultured anaerobically at 37°C for 48 h. Centrifugation was conducted. A resulting bacterial pellet was discarded. A resulting culture supernatant was filtered through a 0.22 µm filter, dispensed, and frozen at -80°C for later use.

Preparation of samples to be tested:
1) Control group: An MM01 liquid medium was diluted with PBS to a volume percentage of 10%.
2) Positive control TSA group: TSA (HDAC inhibitor) was diluted with PBS to a final concentration of 10 µM.
3) MNH19801 group: The MNH19801 culture supernatant was diluted with PBS to a volume percentage of 10%.

HDAC activity assay: 50 µL of a sample to be tested was added to a white 96-well plate, and 50 µL of a reaction reagent prepared according to instructions of a kit was added. Thorough mixing was conducted, and incubation was conducted at 37°C for 30 min. Then, 10 µL of Lysine Developer was added to each reaction well, and thorough mixing was conducted for reaction termination. Incubation was conducted at 37°C for 30 min. Finally, a fluorescence intensity of the sample was measured by a microplate reader with Ex. = 350 nm to 380 nm and Em. = 440 nm to 460 nm. HDAC inhibitory activity analysis: With a fluorescence intensity value of the control group as a baseline of 100%, relative fluorescence intensities of the positive control group and the MNH19801 group were converted into relative HDAC activities.

Results were shown in FIG. 13. MNH19801 could significantly inhibit the activity of HDAC, with a similar inhibitory effect to the HDAC inhibitor TSA (10 µM). Thus, MNH19801 can be used as a substitute for the existing HDAC inhibitor.

Current research results show that HDAC inhibitors exhibit significant anti-tumor effects against tumor cells at a site such as bladder, bone, breast, uterus, central nervous system, esophagus, lung, ovary, pancreas, cholangiocarcinoma, and prostate, and can induce remarkable apoptosis, proliferation inhibition, and cell cycle arrest for such tumor cells. As HDACs work like oncogenes to regulate the onset of cancers, the reduction or inhibition of activities of HDACs has been proved to enable various anti-cancer effects. For example, TSA, as an HDAC inhibitor, can up-regulate the expression of cyclin-dependent kinase inhibitors p21 and p27, down-regulate the contents of cyclin A, cyclin D1, and cyclin D2, and inhibit the activation of CDK, thereby leading to the arrest of tumor cells at the G1 or G2 phase, the retardation of further growth of tumor cells (such as MCF-7 breast cancer cells and HeLa cervical cancer cells), and the promotion of apoptosis of tumor cells (see "Role and Mechanism of Trichostatin A (TSA) in Tumor Immunotherapy", Zhou Yi, Chinese Medicinal Biotechnology). Moreover, TSA can inhibit tumor angiogenesis by down-regulating the expression of tumor angiogenesis-associated genes induced by hypoxia and by directly suppressing the migration and proliferation of endothelial cells. It has also been found in human osteosarcomata that TSA can suppress the expression of hypoxia-inducible factor 1-αlpha (HIF-1α) and vascular endothelial growth factor (VEGF), induce the degradation of the corresponding receptors, and up-regulate the transcription of p53 and VHL, thereby inhibiting tumor angiogenesis (Yang QC, Zeng BF, Shi ZM, et al. Inhibition of hypoxia-induced angiogenesis by trichostatin A via suppression of HIF-1a activity in human osteosarcoma. J Exp Clin Cancer Res, 2006, 25(4):593-599.).

HDAC inhibitors also possess anti-fibrotic effects. Diabetes refers to a group of diseases characterized by hyperglycemia resulting from low insulin levels and/or peripheral insulin resistance. It has been proposed that HDAC inhibitors can treat diabetes through a variety of mechanisms, including inhibition of Pdx1 (Park et al., 2008, J Clin Invest, 118, 2316-24), enhancement of the expression of a transcription factor Ngn3 to expand the pool of endocrine progenitor cells (Haumaitre et al., 2008, Mol Cell Biol, 28, 6373-83), enhancement of the expression of insulin (Molsey et al., 2003, J Biol Chem, 278, 19660-6), etc. The HDAC inhibition is also a promising therapeutic approach for advanced diabetes-associated complications such as diabetic nephropathy and retinal ischemia (Christensen et al., 2011, Mol Med, 17 (5-6), 370-390). Therefore, the composition of the present disclosure can be used for treating or preventing HDAC activity-mediated diabetes.

Studies have also shown that HDAC is essential for mitochondrial adaptation in macrophages through non-histone deacetylation to facilitate IL-1β production, and can enhance the activation of NLRP3-dependent caspase-1 to promote lipopolysaccharide-induced acute inflammation and high-fat diet-induced chronic inflammation. It can be known that the inhibition of HDAC can also be utilized for preventing or treating inflammatory diseases.

Consequently, through an inhibitory effect on the activity of HDAC, the strain provided in the embodiments of the present disclosure can be used for preventing or treating HDAC activity-mediated tumors or cancers, metabolic diseases, inflammatory or autoimmune diseases, infectious diseases, central nervous system diseases, etc. Moreover, due to the crucial roles of HDAC-regulated genes in angiogenesis, this strain can be used to prevent tumor metastasis.

### Example 8: Animal experiment using the strain MNH 19801 for treating liver cancer

To verify whether the strain MNH19801 could be used for the prevention and treatment of tumors, an experiment of inhibiting liver cancer growth was conducted with a syngeneic tumor mouse model. This study had been reviewed and approved by the Management and Use Committee of MoonBiotech.

Test strain: A glycerol stock of the strain MNH 19801 in Example 3 was thawed at 37°C, and then inoculated onto an anaerobic blood agar plate in an anaerobic workstation for activation. An activated strain was inoculated into an MM01 liquid medium and cultured anaerobically to produce a sufficient amount of a culture. The culture was concentrated through centrifugation, and then resuspended in a vehicle (PBS-Cys) to produce a test material with a purity and viable count (2.04 × 10¹⁰ CFU/mL) required by the animal experiment.

Tumor cells: H22 mouse liver cancer cells, CBP69230.

Experimental animals: 20 C57BL/6J mice were adopted for the animal experiment, which were 5 weeks old and purchased from GemPharmatech.

Animal experiment: After a quarantine period under normal raising conditions, the mice were randomly divided into a control group and an experimental group based on body weight, with 10 mice per group. The mice were raised in separate cages. After the grouping, an H22 mouse liver cancer cell suspension with a concentration of 2 × 10⁶ cells/mL was intradermally injected at 0.1 mL/mouse to construct a mouse liver cancer model. Intragastric administration was conducted on the day when the inoculation was completed (DAY 0). The control group was administered with the vehicle. The experimental group was administered with the strain MNH 19801. The intragastric administration was conducted at a dose of 0.2 mL/mouse/time and a frequency of once/day. General observations were conducted once daily during both the quarantine period and the administration period. An animal body weight was measured three times weekly upon animal receipt, at the end of the quarantine period, and during the administration period. Starting from day 7 after tumor cell inoculation, a tumor size was measured three times weekly, and a tumor growth condition was recorded. Endpoint of this animal experiment: The intragastric administration was conducted 19 times. At the experimental endpoint, all mice were euthanized through cervical dislocation. Experimental results were shown in Tables 10 and 11.

**Table 10 Statistics of the overall conditions of mice at the experimental endpoint**

| | Mice with successful tumor engraftment | Mice at the experimental endpoint | Euthanized mice | Average tumor weight (g) | Mean terminal body weight (g) | Mean tumor volume (mm³) |
|---|---|---|---|---|---|---|
| Control group | 7 | 6 | 1 | 2.53 | 30.51 | 2372 |
| Experimental group | 7 | 6 | 1 | 1.8 | 27.88 | 1873 |

**Table 11 Tumor tissue weights after mouse dissection**

| Group | No. | Tumor weight (g) | Mean (g) |
|---|---|---|---|
| Control group | CT1-1 | 2.2896 | 2.52605 |
| | CT1-2 | 5.0875 | |
| | CT2-1 | 1.8192 | |
| | CT2-2 | 0.8667 | |
| | CT3-2 | 3.1895 | |
| | CT3-4 | 1.9038 | |
| Experimental group | A5-16-1 | 0.6565 | 1.8037 |
| | A5-16-3 | 2.0486 | |
| | A5-17-1 | 2.2098 | |
| | A5-17-2 | 2.4457 | |
| | A5-18-2 | 1.8131 | |
| | A5-18-3 | 1.6482 | |

According to the results, tumors in the control group grew rapidly and reached an average weight of approximately 2.53 g on day 19, and tumors in the experimental group had an average weight of about 1.8 g on day 19 and grew significantly slower than the tumors in the control group. This indicates that the strain MNH 19801 can significantly inhibit the growth rate of a liver cancer tumor tissue.

### Example 9: Analysis of immune activation in tumor-bearing mice by the strain MNH19801

### 1. Preparation and cell counting of a spleen single-cell suspension

0.1 g of a spleen tissue was collected from the mice (tumor-bearing mice) at the experimental endpoint in Example 8, rinsed with PBS, homogenized, and resuspended in PBS. A resulting suspension was filtered through a 70 µm filter membrane to produce a mouse spleen single-cell suspension.

A small amount of the mouse spleen single-cell suspension was taken, and a red blood cell lysis buffer, a spleen-counting reagent, pre-cooled PBS, etc. were added successively. A resulting sample was then analyzed by flow cytometry to accurately quantify mouse spleen single cells.

A red blood cell lysis buffer, PBS, etc. were added successively to the remaining mouse spleen single-cell suspension. A resulting cell suspension was then filtered through a 300-mesh filter membrane and then analyzed by flow cytometry.

### 2. Staining of T cells/NK cells among splenocytes

The mouse spleen single-cell suspension was taken, and T-Alexa Fluor647 anti-mouse FOXP3-SP/NK-Alexa Fluor647 anti-mouse FOXP3-SP, T-Live-SP/NK-Live-SP, and T-SP/NK-SP were added to stain T cells/NK cells among splenocytes. Stained cells were resuspended in PBS and analyzed by flow cytometry.

Statistical analysis was performed using one-way ANOVA with Dunnet's multiple comparison: *p < 0.05, **p < 0.01, and ***p < 0.001.

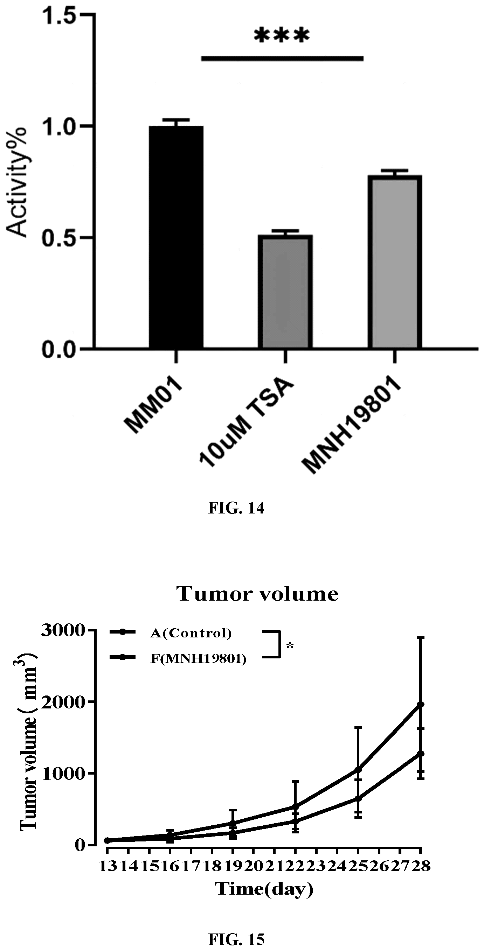

Results were shown in A and B of FIG. 14. Compared with the control group, proportions of CD3⁺ and CD8⁺ cells among mouse splenocytes were significantly up-regulated, indicating increased proportions of T cells and NK cells. Therefore, the strain MNH19801 can activate the systemic immunity of mice by increasing a proportion of T cells/NK cells in the spleen, thereby playing an anti-tumor role.

### Example 10: Animal experiment using the strain MNH 19801 for treating lung cancer

To verify whether the strain MNH19801 could be used for the prevention and treatment of lung cancer, an experiment of inhibiting lung cancer growth was conducted with a syngeneic tumor mouse model. This study had been reviewed and approved by the Management and Use Committee of MoonBiotech.

Test strain: A glycerol stock of the strain MNH 19801 in Example 3 was thawed at 37°C, and then inoculated onto an anaerobic blood agar plate in an anaerobic workstation for activation. An activated strain was inoculated into an MM01 liquid medium and cultured anaerobically to produce a sufficient amount of a culture. The culture was concentrated through centrifugation, and then resuspended in a vehicle (PBS-Cys) to produce a test material with a purity and viable count (1.75 × 10⁸ CFU/mL) required by the animal experiment.

Tumor cells: LLC1 mouse lung cancer cells, CL-0140.

Experimental animals: 24 C57BL/6J mice were adopted for the animal experiment, which were 5 weeks old and purchased from GemPharmatech.

Animal experiment: After a quarantine period under normal raising conditions, an LLC1 lung cancer cell suspension with a concentration of 2 × 10⁶/mL was subcutaneously inoculated at 0.1 mL/mouse to establish an ectopic syngeneic tumor model. When an average tumor volume reached 60 mm³ to 100 mm³, the mice were randomly divided into a control group and an experimental group based on tumor volumes. Intragastric administration was conducted on the day of grouping (Day 1). The control group was administered with the vehicle (PBS-Cys). The experimental group was administered with the strain MNH 19801. The intragastric administration was conducted at a dose of 0.2 mL/mouse/time and a frequency of once/day. General observations were conducted once daily during both the quarantine period and the administration period. An animal body weight was measured three times weekly upon animal receipt, at the end of the quarantine period, and during the administration period. From day 5 to the grouping, a tumor diameter was measured once daily. After the grouping, a tumor diameter was measured once every two days. When an average tumor volume was larger than or equal to 1,000 mm³, a tumor diameter was measured once daily, and a tumor growth condition was recorded. Endpoint of this animal experiment: An average tumor volume in the experimental group was larger than or equal to 1,000 mm³ and was significantly smaller than an average tumor volume in the control group, or average tumor growth inhibition rate (TGI) in the experimental group was higher than or equal to 30%. Alternatively, an average tumor volume in any group exceeded 2,000 mm³. During this animal experiment, general clinical observations, body weight monitoring, and tumor volume (mm³) measuring were conducted. At the experimental endpoint, all surviving mice were dissected for sample collection. A tumor weight was measured, and a tumor volume was calculated. Statistical analysis and comparisons were performed, including a tumor volume change curve, a tumor volume at endpoint, a tumor weight at endpoint, and a tumor growth inhibition rate at endpoint (TGI). Tumor growth inhibition rate (TGI) = 1 - (individual tumor volume - individual tumor volume at grouping)/(average tumor volume of control group - average tumor volume of control group at grouping) × 100%. Data was expressed as mean ± SD. Plotting and statistical analysis were performed using GraphPad Prism 8.0.2 software. For pairwise comparisons, the Student's t-test was employed. For two-factor evaluation, two-way ANOVA (2way ANOVA) with Sidak's multiple comparison was employed. A statistical significance was indicated by *: *p < 0.05, **p < 0.01, ***p < 0.001, and ****p < 0.0001.

### Experimental results were as follows:

**Table 12 Statistics of the overall conditions of mice at the experimental endpoint**

| | Mice with successful tumor engraftment | Mice at the experimental endpoint | Eutha nized mice | Average tumor weight (g) | Mean terminal body weight (g) | Mean tumor volume (mm³) |
|---|---|---|---|---|---|---|
| Control group | 8 | 7 | 1 | 2.475 | 26.46 | 1962.17 |
| Experimental group | 8 | 8 | 0 | 1.769 | 25.06 | 1275.27 |

As shown in FIG. 15, during the animal experiment, a tumor volume of the experimental group was significantly smaller than a tumor volume of the control group. As shown in Table 11, at the experimental endpoint, mice in the control group had an average tumor volume of 1,962.17 mm³ and an average tumor weight of approximately 2.475 g, and mice in the experimental group had an average tumor volume of 1,275.27 mm³ and an average tumor weight of approximately 1.769 g. The mice in the experimental group had a significantly smaller tumor volume and tumor weight than the mice in the control group.

At the experimental endpoint, a tumor growth inhibition rate (TGI) in the experimental group was 36.1% and was significantly higher than a tumor growth inhibition rate in the control group, indicating a substantial tumor growth-inhibiting effect.

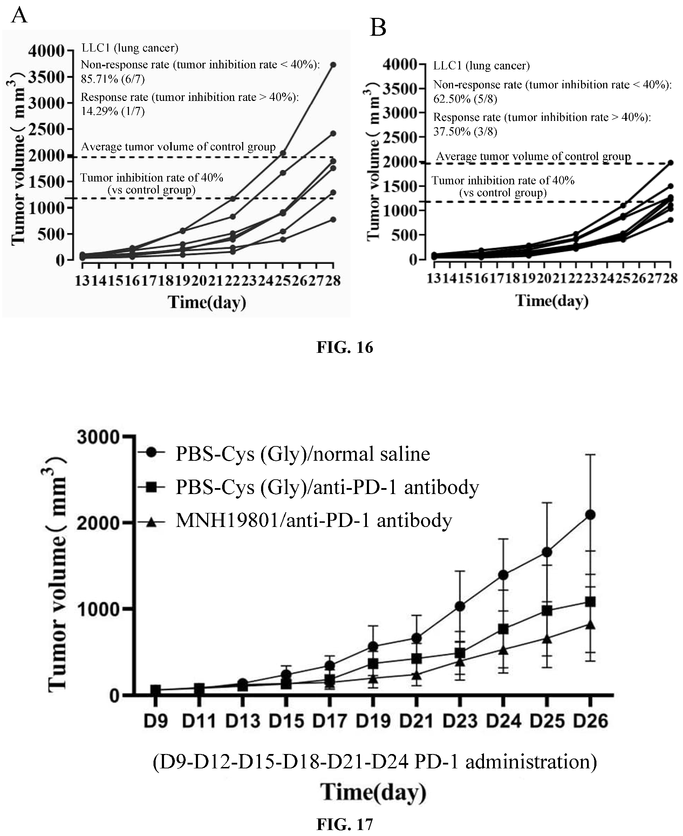

As shown in FIG. 16, at the experimental endpoint, a response rate was 14.29% in the control group and 37.5% in the experimental group. Therefore, the strain MNH19801 can significantly improve the tumor response rate.

### Example 11: Therapeutic effect of the MNH 19801 and PD-1 combonation therapy for lung cancer

In the present disclosure, a plurality of male C57BL/6J mice were purchased. After a quarantine period, tumor cells were inoculated to establish an ectopic syngeneic lung cancer model. The day of tumor cell inoculation was designated as day 1. On day 9, mice with abnormal tumor growth were excluded, and according to tumor volumes, the remaining mice were randomly divided into the following three groups with 8 mice per group: a negative control group, a positive control group (PD-1 group), and a combination therapy group (PD-1 + MNH19801). The negative control group was administered with a vehicle and normal saline. The positive control group was administered with the vehicle and an anti-PD-1 antibody. The combination therapy group was administered with the strain MNH19801 and the anti-PD-1 antibody. The vehicle and MNH19801 were administered intragastrically at a dose of 0.2 mL/mouse/time and a frequency of once/day. The anti-PD-1 antibody and normal saline were intraperitoneally injected at a dose of 10 mg/kg and a frequency of once/3 days. Administration intervention was conducted on the day of grouping according to the groups. During the experiment, an animal condition, a tumor change, a tumor size, and a body weight were regularly monitored. At an experimental endpoint, a body weight change, a tumor volume change, a response rate, and a tumor inhibition rate were analyzed. Throughout the experiment, all mice had a body weight steadily increased.

At the experimental endpoint, compared with the negative control group, both the PD-1 group and the combination therapy group had a tumor volume significantly decreased, and a tumor volume of the combination therapy group was significantly reduced. Compared to the PD-1 group, the tumor volume of the combination therapy group also declined.

As shown in FIG. 17, on day 23 of medication, a tumor volume of mice in the negative control group had a significant difference (p < 0.001, ***) from tumor volumes of mice in the PD-1 group and the combination therapy group, and this difference progressively increased over time. Further, from day 17 of medication to the experimental endpoint on day 28, a tumor volume of mice in the PD-1 group was consistently larger than a tumor volume of mice in the PD-1/MNH19801 combination therapy group. On day 25 of medication, there was a significant difference (p < 0.05, *) in tumor volume between the PD-1 group and the PD-1/MNH19801 combination therapy group.

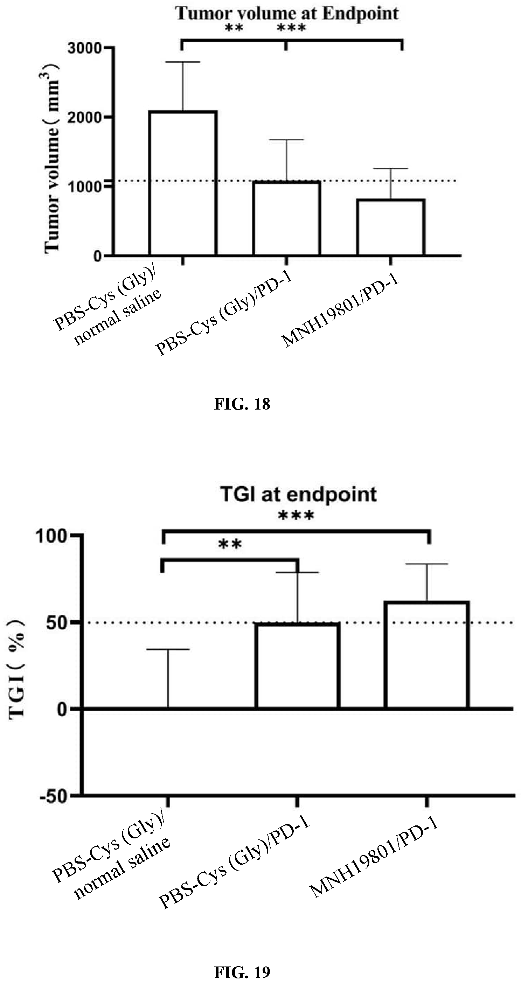

As shown in FIG. 18, at the experimental endpoint, compared with the negative control group, both the PD-1 group and the combination therapy group had a tumor volume significantly decreased, and a tumor volume of the combination therapy group was significantly reduced. Compared to the PD-1 group, a tumor volume of the combination therapy group also significantly declined.

As shown in FIG. 19, at the experimental endpoint, both the PD-1 group and the combination therapy group demonstrated a significant tumor growth inhibition rate (TGI) of higher than or equal to 50% compared with the negative control group, and a tumor growth inhibition rate of the combination therapy group was markedly superior to a tumor growth inhibition rate of the PD-1 group.

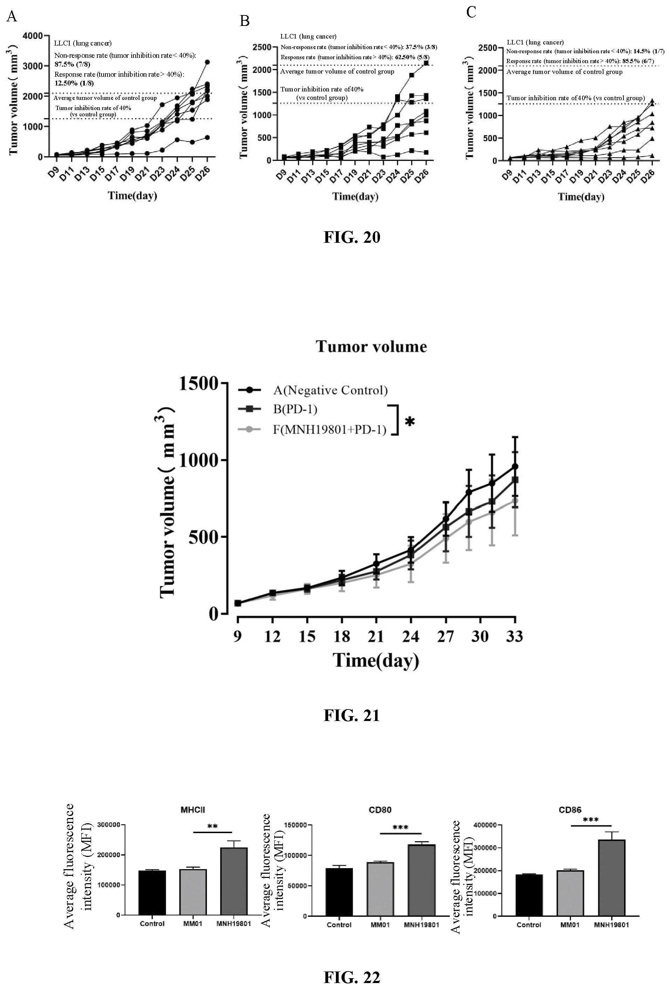

FIGs. 20A-20C show response rates of mice in the groups at the experimental endpoint. Compared with the negative control group, PD-1 response rates in both the PD-1 group and the combination therapy group were significantly increased, and the combination therapy group had a higher PD-1 response rate than the PD-1 group.

In summary, the combination of MNH19801 and PD-1 demonstrates superior therapeutic efficacy against lung cancer, and can effectively suppress the lung cancer growth in mice and enhance the PD-1 response rate.

### Example 12: Therapeutic effect of MNH 19801 and PD-1 combination therapy for pancreatic cancer

In the present disclosure, a plurality of male C57BL/6J mice were purchased. After a quarantine period, mouse pancreatic cancer KPC cells were inoculated to establish an ectopic syngeneic pancreatic cancer model. The day of tumor cell inoculation was designated as day 1. When an average tumor volume reached 60 mm³ to 100 mm³, mice with abnormal tumor growth were excluded, and according to tumor volumes, the remaining mice were randomly divided into the following three groups with 8 mice per group: a negative control group, a positive control group (PD-1 group), and a combination therapy group (PD-1 + MNH19801). The negative control group was administered with a vehicle and normal saline. The positive control group was administered with the vehicle and an anti-PD-1 antibody. The combination therapy group was administered with the strain MNH19801 and the anti-PD-1 antibody. The vehicle and MNH19801 were administered intragastrically at a dose of 0.2 mL/mouse/time and a frequency of once/day. The anti-PD-1 antibody and normal saline were intraperitoneally injected at a dose of 10 mg/kg and a frequency of once/3 days. Administration intervention was conducted on the day of grouping according to the groups. During the experiment, an animal condition, a tumor change, a tumor size, and a body weight were regularly monitored. At an experimental endpoint, a body weight change and a tumor volume change were analyzed. Throughout the experiment, all mice had a body weight steadily increased.

At the experimental endpoint, compared with the negative control group, both the PD-1 group and the combination therapy group had a tumor volume significantly decreased, and a tumor volume of the combination therapy group was significantly reduced. Compared to the PD-1 group, the tumor volume of the combination therapy group also declined.

According to the tumor growth curve in FIG. 21, from day 17 of medication to the experimental endpoint on day 33, a tumor volume of mice in the PD-1 group was consistently larger than a tumor volume of mice in the PD-1/MNH19801 combination therapy group. On day 33 of medication, there was a significant difference (at the experimental endpoint, statistical analysis was performed using Two-Way ANOVA with Dunnett's multiple comparison, *: p < 0.05) in tumor volume between the PD-1 group and the PD-1/MNH19801 combination therapy group.

Thus, the combination of MNH19801 and PD-1 demonstrates better therapeutic efficacy against pancreatic cancer than PD-1 alone, and can suppress the lung cancer growth in mice and enhance the PD-1 response rate.

### Example 13: Evaluation of the influence of the strain MNH19801 on the differentiation and maturation of mouse bone marrow-derived DCs

This example is based on the following principle: DCs play a critical role in immune activation. It has been confirmed that DCs are the only antigen-presenting cells (APCs) capable of significantly stimulating the proliferation of naive T cells. Other types of APCs (such as monocyte-macrophages and B cells) can only stimulate activated or memory T cells. Therefore, DCs are the initiators of the adaptive T cell immune response in the body, and play an extremely critical role in tumor immunity. For mice, the most common method is to induce the generation of DCs from bone marrow cells, namely, bone marrow-derived dendritic cells (BMDCs). Therefore, in this study, with BMDC as a model, biomarkers associated with differentiation and maturation on the surface of DCs (such as CD11c, MHCII, CD80, and CD86) were detected by flow cytometry to evaluate the influence of the strain on the differentiation and maturation of DCs.

Experiment preparation: A DMEM complete medium used in this example was a DMEM medium including 10% of fetal bovine serum (FBS), 1% of penicillin-streptomycin, 1% of L-glutamine, and 1% of an N-2-hydroxyethylpiperazine-N'-2-ethanesulfonic acid (HEPES) buffer (pH: 6.8 to 8.2).

A BMDC induction medium was a DMEM complete medium including 25 ng/mL of granulocyte-macrophage colony-stimulating factor (GM-CSF) and 10 ng/mL of IL-4.

Preparation of an MNH19801 culture supernatant: The strain MNH19801 was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

Control group: DMEM complete medium.

MM01 group: BMDC induction medium including 10% of the MM01 liquid medium.

MNH19801 group: BMDC induction medium including 10% of the MNH19801 culture supernatant.

A BMDC surface marker staining solution was prepared using CD45-AF700, CD11C-PC5.5, MHC-II-APC, CD86-PE-Cy7, and CD80-FITC at 1:100, with 100 µL per reaction system.

Experimental steps: Extraction of mouse bone marrow cells: A femur and a tibia were isolated from a healthy wild-type (C57BL/6) mouse. Two ends of each of the femur and the tibia were cut off. Bone marrow cells were flushed out using a 1 mL syringe filled with pre-cooled PBS, then pipetted up and down for dispersion, ground, and centrifuged at 1,000 rpm for 5 min. Red blood cell lysis was conducted. Resulting cells were resuspended in PBS to produce a single-cell suspension.

Induction and culture of BMDCs: Bone marrow cells were collected through centrifugation, resuspended in 10 mL of a BMDC induction medium, transferred to a 10 cm culture dish, and cultured for 6 d in a 37°C incubator to allow differentiation. During the culturing, a half-medium change was conducted with the BMDC induction medium every two days. After the 6 d culturing, relatively mature BMDCs were obtained.

BMDCs were treated with the culture supernatant. BMDCs were collected through centrifugation, resuspended, counted, and inoculated into a 12-well plate at 1 × 10⁶ cells/well. The cells were treated according to conditions of the control group, the MM01 group, and the MNH19801 group, respectively. After 24 h of culturing, cells were collected through centrifugation, washed with PBS, and stained with the BMDC surface marker staining solution for 30 min. Finally, BMDCs were analyzed by flow cytometry to assess the expression of MHCII, CD80, and CD86 molecules associated with the differentiation and maturation of DCs.

Experimental results: FIG. 22 shows flow cytometry results of molecules MHCII, CD80, and CD86 on the surface of DCs. Compared to the MM01 group, MNH19801 can significantly up-regulate the expression of the MHCII molecule on DCs, and can also markedly promote the up-regulation of CD80 and CD86 molecules. These results indicate that MNH19801 is capable of promoting the differentiation and maturation of DCs and may consequently enhance the activation of anti-tumor immunity.

In FIG. 22, data is expressed as mean ± SD. Statistical analysis is performed using one-way ANOVA with Tukey's multiple comparison. A statistical significance is indicated by *, and significance levels are defined as follows: no significant difference (ns): p ≥ 0.05; *: p < 0.05; **: p < 0.01; ***: p < 0.001; and ****: p < 0.0001.

### Example 14: Evaluation of an inhibitory effect of the strain MNH19801 on M2 tumor associated macrophages (TAMs)

This example is based on the following principle: TAMs play a critical role in the malignant progression of tumors. TAMs can be broadly categorized into M1 and M2 phenotypes. The M1 phenotype exerts a pro-inflammatory effect and inhibit tumor growth. The M2 phenotype shows an anti-inflammatory function, suppresses anti-tumor immune activation, and promote malignant tumor progression. Therefore, for M2 macrophages, an M2 TAM model was constructed by *in vitro* inducing mouse monocytic macrophage leukemia cells (RAW264.7) with IL-4. Expression levels of biomarkers CD206 and Arg1 by M2 macrophages were detected using real-time quantitative PCR to evaluate the influence of the strain on the M2 macrophage phenotype.

Experiment preparation: Preparation of an MNH19801 culture supernatant: The strain MNH19801 was inoculated into an MM01 liquid medium and cultured anaerobically at 37°C for 48 h. Centrifugation was conducted. A resulting bacterial pellet was discarded. A resulting culture supernatant was filtered through a 0.22 µm filter, dispensed, and stored at -80°C for later use.

A DMEM complete medium used in this example included a DMEM medium, FBS, and penicillin-streptomycin (P/S) at 9:1:0.1, for example, 45 mL of the DMEM medium + 5 mL of the FBS + 500 µL of the penicillin-streptomycin (P/S).

Control group: DMEM complete medium including 5% of the MM01 liquid medium.

TAM group: DMEM complete medium including 20 ng/mL of IL4 and 5% of the MM01 liquid medium.

MNH19801 group: DMEM complete medium including 20 ng/mL of IL4 and 5% of the MNH19801 culture supernatant.

After counting, RAW264.7 cells were inoculated into a 6-well plate at 5 * 10⁵/2 mL/well and cultured in a 5% CO₂ and 37°C incubator for 24 h to allow cell adhesion. The next day, the original medium was removed using a pipette, 2 mL of a medium corresponding to the Control, TAM, or MNH19801 was added, and thorough mixing was conducted gently. The 6-well plate was incubated in a 5% CO₂ and 37°C incubator for 2 d. Cells were washed with PBS, and RNA was extracted (TAKARA MiniBEST Universal RNA Extraction Kit, TAKARA) and reverse-transcribed (PrimeScriptTM RT reagent Kit with gDNA Eraser, TAKARA). The expression of CD206, Arg1, and an internal reference gene GAPDH was detected through qPCR (TB Green^{®} Premix Ex Taq^{™} II, TAKARA). All operations were conducted according to instructions for respective kits.

CD206 primers: F-CTCTGTTCAGCTATTGGACGC (SEQ ID NO: 2) and R-CGGAATTTCTGGGATTCAGCTTC (SEQ ID NO: 3).

Arg1 primers: F-CATTGGCTTGCGAGACGTAGAC (SEQ ID NO: 4) and R-GCTGAAGGTCTCTTCCATCACC (SEQ ID NO: 5).

GAPDH primers: F-GTGTTCCTACCCCCAATGTGT (SEQ ID NO: 6) and R-ATTGTCATACCAGGAAATGAGCTT (SEQ ID NO: 7).

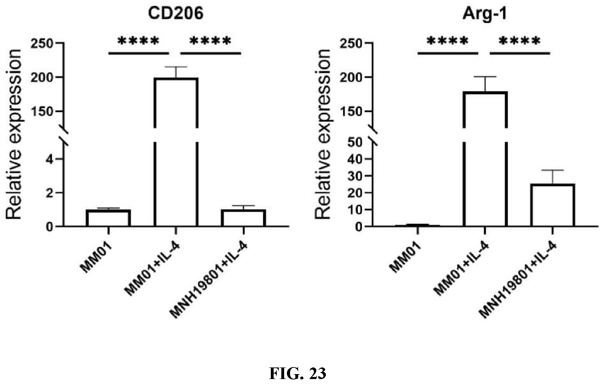

Experimental results: FIG. 23 shows qPCR detection results of the expression of M2 markers CD206 and Arg1 by RAW264.7 cells. According to the results, IL-4 can significantly promote the up-regulation of the expression of M2 macrophage biomarkers CD206 and Arg1, indicating that IL-4 can successfully induce the transition of RAW265.7 cells to the M2 phenotype. The culture supernatant of the strain MNH19801 can significantly suppress the IL-4-induced up-regulation of CD206 and Arg1, indicating that MNH19801 can inhibit the IL-4-induced transition of RAW264.7 cells to the M2 phenotype. These results indicate that MNH26621 possesses the ability to counteract the immunosuppression of M2 macrophages in a tumor microenvironment, thereby promoting the activation of anti-tumor immunity.

In FIG. 23, data is expressed as mean ± SD. Statistical analysis is performed using one-way ANOVA with Tukey's multiple comparison. A statistical significance is indicated by *, and significance levels are defined as follows: no significant difference (ns): p ≥ 0.05; *: p < 0.05; **: p < 0.01; ***: p < 0.001; and ****: p < 0.0001.

The present disclosure has been described in detail above with reference to the examples, but the present disclosure is not limited to the above examples. Within the scope of knowledge possessed by those of ordinary skill in the art, various modifications can be made without departing from the purpose of the present disclosure. In addition, in the case of no conflict, the examples of the present disclosure and the features in the examples may be combined with each other.

## Claims

1. A bacterial strain, wherein the strain is any one of following A1) to A4):
A1) a bacterial strain, wherein the 16S rRNA of the bacterial strain has an identity of at least 95%, 96%, 97%, 98%, 98.5%, 98.65%, 99%, 99.5%, 99.6%, 99.7%, 99.8%, 99.9%, 99.99%, or 100% to the sequence shown in SEQ ID NO: 1;
A2) a bacterial strain MNH19801 with an accession number GDMCC No. 61980;
A3) a bacterial strain, wherein the 16S rRNA has an identity of at least 95%, 96%, 97%, 98%, 98.5%, 98.65%, 99%, 99.5%, 99.6%, 99.7%, 99.8%, 99.9%, 99.99%, or 100% to the 16S rRNA of the strain described in the A2); and
A4) a bacterial strain in which a genome has an average nucleotide identity (ANI) of at least 87%, 90%, 95%, 96%, 97%, 98%, 99%, 99.5%, 99.6%, 99.7%, 99.8%, 99.9%, 99.99%, or 100% with a genome of a strain described in any of A1) to A3); and/or, a strain in which a genome has an alignment fraction of at least 60%, 70%, 80%, 90%, 95%, 96%, 97%, 98%, or 99% with a genome of a strain described in any of the A1) to the A3);
preferably, the sequence of the 16S rRNA of the strain described in the A2) is shown in SEQ ID NO: 1;
preferably, the strain belongs to the family *Lachnospiraceae;*
preferably, the strain belongs to the genus *Blautia* of the family *Lachnospiraceae;*
preferably, the strain is a novel species (*Blautia* sp.) of the genus *Blautia* of the family *Lachnospiraceae;*
preferably, the strain is a novel species (*Oliverpabstia* sp.) of the genus *Oliverpabstia* of the family *Lachnospiraceae;* and
preferably, the strain is capable of producing acetic acid with a high yield.

2. A culture of the strain according to claim 1, wherein
preferably, the culture comprises any one of following C1) to C5):
C1) a fermentation medium for the strain;
C2) a supernatant resulting from the fermentation medium for the strain;
C3) a concentrate of C1) or C2);
C4) an extract of C1) or C2); and
C5) a dried product of any of C1) to C4);
preferably, the fermentation medium is a liquid medium;
preferably, the culture is produced by culturing the strain in the liquid medium under anaerobic culture conditions; and
preferably, the fermentation medium is an MM01 liquid medium, and/or the culturing is conducted at 35°C to 42°C, and/or the culturing is conducted for 24 h to 72 h, and/or a pH of the fermentation medium is 6 to 10.

3. A microbial agent comprising the strain according to claim 1 or a metabolite of the strain according to claim 1 or the culture according to claim 2.

4. A composition, wherein the composition or a raw material for the composition comprises the strain according to claim 1 or the culture according to claim 2 or the microbial agent according to claim 3;
preferably, the composition further comprises at least one of an excipient, a diluent, and a carrier;
preferably, the composition further comprises an adjuvant;
preferably, the adjuvant comprises at least one of a lubricant, a wetting agent, a binder, an emulsifier, a suspension stabilizer, a solubilizing agent, a preservative, a sweetener, and a flavoring agent;
preferably, the composition is at least one of a drug, a nutritional supplement, a food, a food additive, a feed, and a feed additive; and
preferably, the form of the composition is any one of a powder, a granule, a tablet, a capsule, a gel, a suspension, a drop, a drop pill, an injection, a suppository, an aerosol, an oral liquid, an ointment, an emulsion, and an irrigation solution.

5. A pharmaceutical composition comprising following D1) and D2):
D1) the strain according to claim 1 or a metabolite of the strain according to claim 1 or the culture according to claim 2 or the microbial agent according to claim 3; and
D2) a drug for combination therapy,
wherein preferably, the drug for combination therapy comprises at least one of an anti-tumor drug, an anti-metabolic disease drug, an anti-inflammatory disease drug, an anti-autoimmune disease drug, an anti-infectious disease drug, and an anti-central nervous system disease drug;
preferably, the pharmaceutical composition further comprises an instruction for use;
preferably, the anti-tumor drug comprises at least one of a chemotherapeutic drug, a photosensitizer, a photothermal therapy agent, and an immunotherapeutic drug;
preferably, the chemotherapeutic drug comprises at least one of paclitaxel, camptothecin, 5-fluorouracil, cisplatin, doxorubicin, mitomycin, and epirubicin;
preferably, the photosensitizer comprises at least one of BODIPY, chlorin, and rose bengal;
preferably, the photothermal therapy agent comprises at least one of a noble metal nanoparticle, an organic polymer, a carbon-based nanomaterial, a magnetic nanomaterial, and a semiconductor nanomaterial;
preferably, the immunotherapeutic drug comprises at least one of an immune cell therapy drug and an immune checkpoint inhibitor;
preferably, the immune cell therapy drug comprises at least one of a T cell therapy drug, a tumor-infiltrating lymphocyte therapy drug, and a natural killer (NK) cell therapy drug;
preferably, the immune checkpoint inhibitor comprises an inhibitor targeting at least one of programmed cell death protein 1 (PD-1), programmed cell death ligand 1 (PD-L1), programmed cell death ligand 2 (PD-L2), cytotoxic T-lymphocyte-associated protein 4 (CTLA-4), T-cell immunoglobulin (Ig) and mucin-domain containing protein 3 (TIM-3), lymphocyte-activation gene 3 (LAG-3), T-cell immunoreceptor with Ig and immunoreceptor tyrosine-based inhibitory motif (ITIM) domain (TIGIT), V-domain Ig suppressor of T cell activation (VISTA), and B and T lymphocyte attenuator (BTLA);
preferably, the pharmaceutical composition comprises 1 × 10³ CFU/g to 1 × 10¹³ CFU/g of the strain;
preferably, the pharmaceutical composition comprises at least 1 × 10³ CFU/g, 2 × 10³ CFU/g, 5 × 10³ CFU/g, 1 × 10⁴ CFU/g, 2 × 10⁴ CFU/g, 5 × 10⁴ CFU/g, 1 × 10⁵ CFU/g, 2 × 10⁵ CFU/g, 5 × 10⁵ CFU/g, 1 × 10⁶ CFU/g, 2 × 10⁶ CFU/g, 5 × 10⁶ CFU/g, 1 × 10⁷ CFU/g, 2 × 10⁷ CFU/g, 5 × 10⁷ CFU/g, 1 × 10⁸ CFU/g, 2 × 10⁸ CFU/g, 5 × 10⁸ CFU/g, 1 × 10⁹ CFU/g, 2 × 10⁹ CFU/g, 5 × 10⁹ CFU/g, 1 × 10¹⁰ CFU/g, 2 × 10¹⁰ CFU/g, 5 × 10¹⁰ CFU/g, 1 × 10¹¹ CFU/g, 2 × 10¹¹ CFU/g, 5 × 10¹¹ CFU/g, 1 × 10¹² CFU/g, 2 × 10¹² CFU/g, 5 × 10¹² CFU/g, or 1 × 10¹³ CFU/g of the strain;
preferably, the strain in the pharmaceutical composition comprises a live bacterium, an attenuated bacterium, a killed bacterium, a lyophilized bacterium, or an irradiated bacterium;
preferably, the pharmaceutical composition or a raw material for the pharmaceutical composition comprises the D1) in a mass percentage of 1% to 80% or 40% to 60%;
preferably, the pharmaceutical composition or the raw material for the pharmaceutical composition comprises the D1) in a mass percentage of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, or 80%;
preferably, the pharmaceutical composition or the raw material for the pharmaceutical composition comprises the D1) in a mass percentage of 1% to 80%, 2% to 70%, 5% to 60%, 10% to 50%, or 20% to 40%;
preferably, the pharmaceutical composition further comprises at least one of an excipient, a diluent, and a carrier;
preferably, the pharmaceutical composition further comprises a pharmaceutically acceptable adjuvant;
preferably, the pharmaceutically acceptable adjuvant comprises at least one of a lubricant, a wetting agent, an emulsifier, a suspension stabilizer, a preservative, a sweetener, and a flavoring agent;
preferably, the pharmaceutical composition is in an infant-suitable dosage form, a child-suitable dosage form, or an adult-suitable dosage form;
preferably, the pharmaceutical composition is in a dosage form for enteral administration or a dosage form for parenteral administration;
preferably, the pharmaceutical composition is an oral preparation or an injection;
preferably, a dosage form of the pharmaceutical composition is any one of a powder, a granule, a tablet, a capsule, a gel, a suspension, a drop, a drop pill, an injection, a suppository, an aerosol, an oral liquid, an ointment, an emulsion, and an irrigation solution; and
preferably, the strain is capable of at least partially proliferating in an intestine of a subject.

6. A pharmaceutical combination product, comprising the following independent components:
a first product comprising the strain according to claim 1 or a metabolite of the strain according to claim 1 or the culture according to claim 2 or the microbial agent according to claim 3; and
a second product comprising a drug for combination therapy,
wherein preferably, the drug for combination therapy comprises at least one of an anti-tumor drug, an anti-metabolic disease drug, an anti-inflammatory disease drug, an anti-autoimmune disease drug, an anti-infectious disease drug, and an anti-central nervous system disease drug;
preferably, the pharmaceutical combination product further comprises a package insert;
preferably, the anti-tumor drug comprises at least one of a chemotherapeutic drug, a photosensitizer, a photothermal therapy agent, and an immunotherapeutic drug;
preferably, the chemotherapeutic drug comprises at least one of paclitaxel, camptothecin, 5-fluorouracil, cisplatin, doxorubicin, mitomycin, and epirubicin;
preferably, the photosensitizer comprises at least one of BODIPY, chlorin, and rose bengal;
preferably, the photothermal therapy agent comprises at least one of a noble metal nanoparticle, an organic polymer, a carbon-based nanomaterial, a magnetic nanomaterial, and a semiconductor nanomaterial;
preferably, the immunotherapeutic drug comprises at least one of an immune cell therapy drug and an immune checkpoint inhibitor;
preferably, the immune cell therapy drug comprises at least one of a T cell therapy drug, a tumor-infiltrating lymphocyte therapy drug, and an NK cell therapy drug;
preferably, the immune checkpoint inhibitor comprises an inhibitor targeting at least one of PD-1, PD-L1, PD-L2, CTLA-4, TIM-3, LAG-3, TIGIT, VISTA, and BTLA;
preferably, the first product comprises 1 × 10³ CFU/g to 1 × 10¹³ CFU/g of the strain;
preferably, the first product comprises at least 1 × 10³ CFU/g, 2 × 10³ CFU/g, 5 × 10³ CFU/g, 1 × 10⁴ CFU/g, 2 × 10⁴ CFU/g, 5 × 10⁴ CFU/g, 1 × 10⁵ CFU/g, 2 × 10⁵ CFU/g, 5 × 10⁵ CFU/g, 1 × 10⁶ CFU/g, 2 × 10⁶ CFU/g, 5 × 10⁶ CFU/g, 1 × 10⁷ CFU/g, 2 × 10⁷ CFU/g, 5 × 10⁷ CFU/g, 1 × 10⁸ CFU/g, 2 × 10⁸ CFU/g, 5 × 10⁸ CFU/g, 1 × 10⁹ CFU/g, 2 × 10⁹ CFU/g, 5 × 10⁹ CFU/g, 1 × 10¹⁰ CFU/g, 2 × 10¹⁰ CFU/g, 5 × 10¹⁰ CFU/g, 1 × 10¹¹ CFU/g, 2 × 10¹¹ CFU/g, 5 × 10¹¹ CFU/g, 1 × 10¹² CFU/g, 2 × 10¹² CFU/g, 5 × 10¹² CFU/g, or 1 × 10¹³ CFU/g of the strain;
preferably, the strain in the first product comprises a live bacterium, an attenuated bacterium, a killed bacterium, a lyophilized bacterium, or an irradiated bacterium;
preferably, the first product and/or the second product further comprises at least one of an excipient, a diluent, and a carrier;
preferably, the first product and the second product independently further comprise at least one pharmaceutically acceptable adjuvant;
preferably, the at least one pharmaceutically acceptable adjuvant comprises at least one of a lubricant, a wetting agent, an emulsifier, a suspension stabilizer, a preservative, a sweetener, and a flavoring agent;
preferably, the pharmaceutical combination product is in an infant-suitable dosage form, a child-suitable dosage form, or an adult-suitable dosage form;
preferably, the first product and the second product are each independently in a dosage form for enteral administration or a dosage form for parenteral administration;
preferably, the first product and the second product are each independently an oral preparation or an injection;
preferably, the first product and the second product are each independently any one of a powder, a granule, a tablet, a capsule, a gel, a suspension, a drop, a drop pill, an injection, a suppository, an aerosol, an oral liquid, an ointment, an emulsion, and an irrigation solution; and
preferably, the strain is capable of at least partially proliferating in an intestine of a subject.

7. Use of the strain according to claim 1, the culture according to claim 2, the microbial agent according to claim 3, the composition according to claim 4, the pharmaceutical composition according to claim 5, or the pharmaceutical combination product according to claim 6 in preparation of a drug for preventing and/or treating a disease,
wherein preferably, the disease comprises at least one of a tumor or cancer, a metabolic disease, an inflammatory or autoimmune disease, an infectious disease, and a central nervous system disease;
preferably, the tumor or cancer comprises at least one of a solid tumor, a soft tissue tumor, a hematopoietic tumor, a glandular tumor, and a metastatic tumor;
preferably, the solid tumor comprises at least one of head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer;
preferably, the hematopoietic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus;
preferably, the bacterium or virus comprises at least one of hepatitis B virus, hepatitis C virus, human papillomavirus, Epstein-Barr (EB) virus, *Helicobacter pylori,* and *Fusobacterium nucleatum;*
preferably, the metabolic disease comprises a disease **characterized by** a metabolic disorder in at least one of an amino acid, an organic acid, a sugar, a fat, a purine, a pigment, blood ammonia, and a metal ion;
preferably, the inflammatory or autoimmune disease comprises at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome;
preferably, the infectious disease comprises a disease caused by an infection with at least one of a bacterium, a virus, and a fungus;
preferably, the central nervous system disease comprises at least one of stroke, schizophrenia, drug addiction, Alzheimer's disease, Parkinson's disease, anxiety disorder, and depression; and
preferably, preventing and/or treating a tumor is achieved through at least one of following (a) to (l):
(a) inhibiting an increase in tumor volume;
(b) inhibiting an increase in tumor weight;
(c) inhibiting proliferation of a tumor cell;
(d) increasing a response rate of a tumor treatment;
(e) enhancing therapeutic efficacy of an immunosuppressive drug, comprising improving therapeutic efficacy of a PD-1 inhibitor;
(f) preventing and/or inhibiting dissemination or metastasis of a tumor cell;
(g) reducing resistance to an anti-tumor drug;
(h) inhibiting an activity of a histone deacetylase (HDAC) through at least one of short-chain fatty acids (SCFAs) comprising acetic acid or acetate, propionic acid or propionate, butyric acid or butyrate, and valeric acid or valerate;
(i) inhibiting an activity of HDAC and/or promoting a transcriptional activity of interferon-β (IFN-β);
(j) modulating an immune system of a subject to play an immunomodulatory role;
(k) promoting senescence or apoptosis of a tumor cell; and
(l) inhibiting angiogenesis within a tumor tissue.

8. Use of the strain according to claim 1, the culture according to claim 2, the microbial agent according to claim 3, the composition according to claim 4, the pharmaceutical composition according to claim 5, or the pharmaceutical combination product according to claim 6 in prevention and/or treatment of a disease,
wherein preferably, the disease comprises at least one of a tumor or cancer, a metabolic disease, an inflammatory or autoimmune disease, an infectious disease, and a central nervous system disease;
preferably, the tumor or cancer comprises at least one of a solid tumor, a soft tissue tumor, a hematopoietic tumor, a glandular tumor, and a metastatic tumor;
preferably, the solid tumor comprises at least one of head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer;
preferably, the hematopoietic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus;
preferably, the bacterium or virus comprises at least one of hepatitis B virus, hepatitis C virus, human papillomavirus, EB virus, *Helicobacter pylori,* and *Fusobacterium nucleatum;*
preferably, the metabolic disease comprises a disease **characterized by** a metabolic disorder in at least one of an amino acid, an organic acid, a sugar, a fat, a purine, a pigment, blood ammonia, and a metal ion;
preferably, the inflammatory or autoimmune disease comprises at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, Behcet's disease, asthma, atopic dermatitis, acne, Crohn's disease, ulcerative colitis, bronchitis, allergic rhinitis, Graves' disease, Hashimoto's thyroiditis, rheumatoid arthritis, type 1 diabetes, systemic lupus erythematosus, scleroderma, glomerulonephritis, systemic vasculitis, Addison's disease, autoimmune myositis, and Sjogren's syndrome;
preferably, the infectious disease comprises a disease caused by an infection with at least one of a bacterium, a virus, and a fungus;
preferably, the central nervous system disease comprises at least one of stroke, schizophrenia, drug addiction, Alzheimer's disease, Parkinson's disease, anxiety disorder, and depression; and
preferably, preventing and/or treating a tumor is achieved through at least one of following (a) to (l):
(a) inhibiting an increase in tumor volume;
(b) inhibiting an increase in tumor weight;
I inhibiting proliferation of a tumor cell;
(d) increasing a response rate of a tumor treatmenI(e) enhancing therapeutic efficacy of an immunosuppressive drug, comprising improving therapeutic efficacy of a PD-1 inhibitor;
(f) preventing and/or inhibiting dissemination or metastasis of a tumor cell;
(g) reducing resistance to an anti-tumor drug;
(h) inhibiting an activity of HDAC through at least one of SCFAs comprising acetic acid or acetate, propionic acid or propionate, butyric acid or butyrate, and valeric acid or valerate;
(i) inhibiting an activity of HDAC and/or promoting a transcriptional activity of IFN-β;
(j) modulating an immune system of a subject to play an immunomodulatory role;
(k) promoting senescence or apoptosis of a tumor cell; and
(l) inhibiting angiogenesis within a tumor tissue.

9. A method for treating a disease, comprising administering an effective dose of the strain according to claim 1, the culture according to claim 2, the microbial agent according to claim 3, the composition according to claim 4, the pharmaceutical composition according to claim 5, or the pharmaceutical combination product according to claim 6 to a subject,
wherein preferably, the disease comprises at least one of a tumor or cancer, a metabolic disease, an inflammatory or autoimmune disease, an infectious disease, and a central nervous system disease;
preferably, the tumor or cancer comprises at least one of a solid tumor, a soft tissue tumor, a hematopoietic tumor, a glandular tumor, and a metastatic tumor;
preferably, the solid tumor comprises at least one of head and neck cancer, upper gastrointestinal cancer, lower gastrointestinal cancer, hepatobiliary system cancer, neuroendocrine tumor, thoracic cancer, bone tumor, soft tissue tumor, skin cancer, breast cancer, female reproductive system cancer, male reproductive system cancer, urinary system cancer, eye cancer, central nervous system cancer, and endocrine system cancer;
preferably, the hematopoietic tumor comprises a tumor in at least one of blood, lymph, and bone marrow;
preferably, the tumor or cancer comprises at least one of oral cancer, salivary gland cancer, nasopharyngeal cancer, oropharyngeal cancer, hypopharyngeal cancer, nasal cavity cancer, paranasal sinus cancer, laryngeal cancer, esophageal cancer, gastroesophageal junction cancer, gastric cancer, small intestine cancer, appendix cancer, colon cancer, rectal cancer, anal canal cancer, liver cancer, intrahepatic cholangiocarcinoma, gallbladder cancer, hilar cholangiocarcinoma, distal cholangiocarcinoma, ampullary cancer, pancreatic cancer, gastric neuroendocrine tumor, duodenal and ampullary neuroendocrine tumor, jejuno-ileal neuroendocrine tumor, appendiceal neuroendocrine tumor, colorectal neuroendocrine tumor, pancreatic neuroendocrine tumor, thymic carcinoma, lung cancer, malignant pleural mesothelioma, angiosarcoma, desmoid tumor, Ewing sarcoma, fibrosarcoma, gastrointestinal stromal tumor, Kaposi sarcoma, leiomyosarcoma, liposarcoma, myxofibrosarcoma, malignant peripheral nerve sheath tumor, rhabdomyosarcoma, synovial sarcoma, undifferentiated pleomorphic sarcoma, dermatofibrosarcoma protuberans, Merkel cell carcinoma, cutaneous malignant melanoma, vulvar cancer, vaginal cancer, cervical cancer, endometrial carcinoma, uterine carcinosarcoma, uterine sarcoma, ovarian cancer, fallopian tube cancer, primary peritoneal cancer, gestational trophoblastic neoplasia, penile cancer, prostate cancer, testicular cancer, kidney cancer, renal pelvis cancer, ureteral cancer, bladder cancer, urethral cancer, eyelid carcinoma, conjunctival carcinoma, conjunctival melanoma, uveal melanoma, retinoblastoma, lacrimal gland cancer, orbital sarcoma, ocular adnexal lymphoma, brain cancer, spinal cord tumor, differentiated thyroid cancer, anaplastic thyroid cancer, medullary thyroid cancer, parathyroid cancer, adrenocortical carcinoma, Hodgkin lymphoma, non-Hodgkin lymphoma, cutaneous lymphoma, plasma cell myeloma, and leukemia;
preferably, the tumor or cancer comprises a tumor or cancer caused by a bacterium or virus;
preferably, the bacterium or virus comprises at least one of hepatitis B virus, hepatitis C virus, human papillomavirus, EB virus, *Helicobacter pylori,* and *Fusobacterium nucleatum;*
preferably, the metabolic disease comprises a disease **characterized by** a metabolic disorder in at least one of an amino acid, an organic acid, a sugar, a fat, a purine, a pigment, blood ammonia, and a metal ion;
preferably, the inflammatory or autoimmune disease comprises at least one of psoriasis, psoriatic arthritis, multiple sclerosis, inflammatory bowel disease, ankylosing spondylitis, chronic obstructive pulmonary disease, glomerulonephritis, myocarditis, dry eye syndrome, uveitis, 'ehcet's disease, asthma, atopic dermatitis, acne,'Crohn's disease, ulcerative colitis, bronchitis, allergic rhinitis, 'raves' disease, Has'imoto's thyroiditis, rheumatoid arthritis, type 1 diabetes, systemic lupus erythematosus, scleroderma, glomerulonephritis, systemic vasculitis, A'dison's disease, autoimmune myositis, and S'ogren's syndrome;
preferably, the infectious disease comprises a disease caused by an infection with at least one of a bacterium, a virus, and a fungus;
preferably, the central nervous system disease comprises at least one of stroke, schizophrenia, drug addiction, Alz'eimer's disease, Par'inson's disease, anxiety disorder, and depression;
preferably, preventing and/or treating a tumor is achieved through at least one of following (a) to (l):
(a) inhibiting an increase in tumor volume;
(b) inhibiting an increase in tumor welt;
(c) inhibiting proliferation of a tumor cell;
(d) increasing a response rate of a tumor trIment;
(e) enhancing therapeutic efficacy of an immunosuppressive drug, comprising improving therapeutic efficacy of a PD-1 inhibitor;
(f) preventing and/or inhibiting dissemination or metastasis of a tumor cell;
(g) reducing resistance to an anti-tumor drug;
(h) inhibiting an activity of HDAC through at least one of SCFAs comprising acetic acid or acetate, propionic acid or propionate, butyric acid or butyrate, and valeric acid or valerate;
(i) inhibiting an activity of HDAC and/or promoting a transcriptional activity of IFN-β;
(j) modulating an immune system of a subject to play an immunomodulatory role;
(k) promoting senescence or apoptosis of a tumor cell; and
(l) inhibiting angiogenesis within a tumor tissue;
preferably, the strain, the culture, the microbial agent, the composition, the pharmaceutical composition, or the pharmaceutical combination product is administered at least once daily;
preferably, when the strain, the culture, the microbial agent, the composition, the pharmaceutical composition, or the pharmaceutical combination product is administered twice or more daily, the strain, the culture, the microbial agent, the composition, the pharmaceutical composition, or the pharmaceutical combination product is administered at a same dose;
preferably, when the strain, the culture, the microbial agent, the composition, the pharmaceutical composition, or the pharmaceutical combination product is administered twice or more daily, the strain, the culture, the microbial agent, the composition, the pharmaceutical composition, or the pharmaceutical combination product is administered at different doses; and
preferably, a route for the administering comprises at least one of oral administration, sublingual administration, nasal administration, rectal administration, inhalation administration, transdermal administration, intraperitoneal injection, subcutaneous injection, and intramuscular injection.

10. A method for preparing the culture according to claim 2, comprising the following step:
inoculating the strain according to claim 1 into a fermentation medium to produce the culture,
wherein preferably, the method further comprises centrifuging the culture and collecting a supernatant resulting from the fermentation medium;
preferably, the method further comprises concentrating the fermentation medium or the supernatant resulting from the fermentation medium through at least one of evaporation, lyophilization, dialysis, extraction, and membrane separation to produce the concentrate;
preferably, the method further comprises subjecting the fermentation medium or the supernatant resulting from the fermentation medium to at least one of extraction and solvent extraction to produce the extract; and
preferably, the method further comprises drying any one of the fermentation medium, the supernatant resulting from the fermentation medium, the concentrate, and the extract to produce the dried product.
